# EUROPEAN PATENT APPLICATION

(11) **EP 4 603 586 A1**
(43) Date of publication of application: **20.08.2025**
(21) Application number: 24158154.5
(22) Date of filing: 16.02.2024
(51) Int. Cl.: C12N 15/113, A61K 31/7088, A61F 2/06

(54) **INHIBITORY OLIGONUCLEOTIDES AGAINST ADAMTS7**

(71) Applicant: Universität zu Lübeck, 23562 Lübeck (DE)
(72) Inventor: Aherrahrou, Zouhair, 23562 Lübeck (DE); Erdmann, Jeanette, deceased (DE)
(74) Representative: Doll, Markus Alexander

(57) **Abstract**

The present invention relates to the field of therapies against cardiovascular diseases. In particular, the invention provides inhibitory oligonucleotides capable of selectively binding to specific target nucleic acid sequences within the mRNA encoding *ADAMTS7.* The inhibitory oligonucleotides may be RNA molecules capable of inducing RNAi, such as siRNAs or shRNA, or antisense oligonucleotides. In further aspects, the invention relates to different compositions providing these oligonucleotides. These compositions may either comprise the *in* vitro-produced inhibitory oligonucleotides or they may comprise a delivery vehicle, e.g., a viral vector, configured to express the inhibitory oligonucleotides in situ. The invention further provides an implantable medical device, such as, e.g., a stent or a balloon catheter, that comprises and can release the inhibitory oligonucleotides or compositions of the invention. The different inhibitory oligonucleotides, compositions and medical devices can be used for treating different cardiovascular diseases and disorders as well as inflammatory or pulmonary diseases.

## Description

The present invention relates to the field of therapies against cardiovascular diseases. In particular, the invention provides inhibitory oligonucleotides capable of selectively binding to specific target nucleic acid sequences within the mRNA encoding *ADAMTS7.* The inhibitory oligonucleotides may be RNA molecules capable of inducing RNAi, such as siRNAs or shRNA, or antisense oligonucleotides. In further aspects, the invention relates to different compositions providing these oligonucleotides. These compositions may either comprise the *in* vitro-produced inhibitory oligonucleotides or they may comprise a delivery vehicle, e.g., a viral vector, configured to express the inhibitory oligonucleotides in situ. The invention further provides an implantable medical device, such as, e.g., a stent or a balloon catheter, that comprises and can release the inhibitory oligonucleotides or compositions of the invention. The different inhibitory oligonucleotides, compositions and medical devices can be used for treating different cardiovascular diseases and disorders as well as inflammatory or pulmonary diseases.

Atherosclerosis is a chronic disease of the large and medium-sized arteries, which is clinically manifested by narrowing of the vessel lumen and formation of thrombi (Libby et al., 2011; Lusis, 2000; Virmani et al., 2000). Atherogenesis is characterized by the deposition of cholesterol- and apolipoprotein B-containing lipoproteins in the arterial intima (Ference et al, 2017; Libby et al., 2011). The most important apolipoprotein B-containing lipoprotein is low-density lipoprotein (LDL), which is measured in clinical chemistry as LDL cholesterol (LDL-C) (Ference et al., 2017). LDL has a high cholesterol content of approx. 45 % and contains the apolipoprotein B-100 (Rassow, 2012).

Probably the most significant secondary disease of artherosclerosis is known as coronary artery disease (CAD), which currently represents the leading cause of death in the developed world (Naghavi et al, 2017). Due to the narrowing of the vessel lumen as a consequence of atherosclerotic deposits, the supply of oxygen-rich blood to affected tissues becomes limited, with clinical consequences including development of chest pain and myocardial infarction. Despite stent intervention and treatment of classical risk factors such as hypercholesteremia and hypertension, CAD remains the most prevalent cardiovascular disorder (Roth et al., 2020, Kim and Dean, 2011). Heritability estimates for CAD vary between 40% to 70%, suggesting a strong genetic contribution to disease pathology (McPherson and Tybjaerg-Hansen, 2016).

Statins, e.g., atorvastatin, fluvastatin, lovastatin, pitavastatin, pravastatin, rosuvastain or simvastatin, have been used as standard treatment against atherosclerosis and CAD for years. Statins belong to the substance class of 3-hydroxy-3-methylglutaryl coenzyme A reductase (HMG-CoA reductase). Statins lower the level of LDL cholesterol by competitively inhibiting HMG-CoA reductase, the rate-limiting enzyme of the so-called mevalonate pathway. Since statins have a similar structure to HMG-CoA at the molecular level, they fit into the active site of the enzyme and compete with the native substrate (HMG-CoA). This competition reduces the rate at which HMG-CoA reductase can produce mevalonate, a molecule from which cholesterol is ultimately formed.

After a long period of stagnation since the discovery of statins as the modern drug of choice to lower lipid levels in patients with hyperlipidemia, new therapies for CAD have been developed in recent years. Examples include so-called PCSK9 inhibitors. The serine protease PCSK9 binds to LDL receptors on the surface of hepatocytes, thereby promoting their degradation in lysosomes. PCSK9 inhibitors prevent PCSK9 from docking to LDL receptors so that, as a result, the degradation of LDL receptors is reduced. In consequence, LDL receptor concentrations in the cell membrane increase, which leads to a reduction of LDL cholesterol (LDL-C) in the blood, as it is increasingly absorbed into the liver cells. Conventional PCSK9 inhibitors are antibodies that can selectively bind to circulating PCSK9 in the patient's body and thereby block its interaction with the LDL receptor. However, recently, a new type of PCSK9 inhibitor, Inclisiran, received FDA approval. Inclisiran therapy is based on a non-viral delivery of small interfering RNA (siRNA) targeting PCSK9 (Pecin et al., 2017, Ge et al., 2021). Inclisiran thus prevents production of PCSK9 by cells.

However, all current therapies are aiming to lower lipid levels in CAD patients, but do not address underlying primary processes such as inflammation as well as cell proliferation, migration and adhesion. All these processes are well-known to play key roles in plaque progression and rupture independently from hypercholesteremia. Accordingly, there is a significant need to fill this gap by identifying novel and effective therapies against CAD and related diseases.

This problem is solved by the present invention, in particular by the subject matter of the claims.

In particular, the present invention provides inhibitory oligonucleotides capable of selectively binding to a target nucleic acid sequence within an mRNA encoding *ADAMTS7,* wherein the target nucleic acid sequence is any of SEQ ID NO: 1, SEQ ID NO: 2, SEQ ID NO:3, SEQ ID NO: 4, SEQ ID NO: 5, SEQ ID NO: 6 and SEQ ID NO: 7 and wherein the inhibitory oligonucleotides are either RNA molecules capable of inducing RNAi or antisense oligonucleotides (ASOs).

In a quest to develop novel drugs against CAD, significant efforts were made over the last decade to identify genetic loci within the human genome that are significantly associated with CAD using genome-wide association studies (GWAS) (Erdmann et al., 2018, Erdmann et al., 2011, Schunkert et al, 2011, Myocardial Infarction Genetics Consortium et al., 2009, Erdmann et al., 2009, Samani et al., 2007).

Among the identified GWAS CAD loci, one locus harbors the *ADAMTS7* gene. ADAMTS7 (A Disintegrin And Metalloproteinase with ThromboSpondin motifs, seven) protein acts as an ECM protease enzyme. ECM enzymes involve various physiological functions, including coagulation, inflammation, arthritis, angiogenesis, and cell migration. The present inventors' GWAS finding of 2011 relating ADAMTS7 to CAD (Schunkert et al., 2011), has generated an increased focus on ADAMTS7 and its pathophysiological role in atherosclerosis. Various clinical studies and investigations in mice demonstrate that ADAMTS7 is associated with a high risk of atherosclerosis and restenosis (Schunkert et al., 2011, Wang et al., 2009, Qin et al., 2017, Kessler et al., 2015, van Setten et al., 2013) , and that mice lacking ADAMTS7 exhibit reduced atherosclerosis and neointima formation (Kessler et al., 2015, Bauer et al., 2015).

For instance, Kessler et al., 2015 generated mice lacking ADAMTS7 (Adamts7 KO) by inserting an internal ribosome entry site followed by the β-galactosidase sequence between exons 4 and 5. The authors of this study used wire injury in the carotid artery of these Adamts7 Knockout (KO) mice and WT littermate mice and tested for endothelialization at early stages (3, 5, and 7 days) post-injury followed by the examination of neo-intima formation at 14 and 28 days. Endothelial cells (ECs) from WT mice were severely damaged immediately after injury and recovered slowly over time. Furthermore, moderate neointimal hyperplasia could be observed in WT mice. By contrast, re-endothelialization in Adamts7 KO mice was much faster in the early stages, and subsequent neointimal hyperplasia was wholly absent at 14 and 28 days.

Extensive phenotyping of the *Adamts7* KO mice and control wild-type littermates (WT), including measurements of vital parameters, echocardiographic analysis, severe organ dysfunction, or histopathologic abnormalities, demonstrated that lack of ADAMTS7 did not lead to any obvious abnormal phenotypes in mice (Kessler et al., 2015), suggesting that therapeutically targeting ADAMTS7 is a safe approach for treating atherosclerosis and CAD. In support of this, recent analysis of gnomAD data (available at gnomad.broadinstitute.org) provided intriguing findings regarding rare mutations affecting ADAMTS7. This study identified rare human individuals who carry mutations resulting in the loss of function of ADAMTS7, yet remarkably, these individuals remain in good health.

Thus, based on clinical and mouse data, the present inventors concluded that ADAMTS7 can be a potential target for treatment of CAD.

In the context of the present invention, the term "oligonucleotide" is herein understood to refer to a short polymeric sequence of typically RNA or DNA nucleotides. Oligonucleotides may have a minimum length of 2 nucleotides (nt) and a maximum length of about 100 nt. However, typical oligonucleotides consist of about 12-25 nt. An "inhibitory oligonucleotide" is herein understood as a type of oligonucleotide that is capable of post-transcriptionally reducing or even completely preventing the expression of a gene of interest into a protein by selectively binding to a target sequence within an mRNA expressed from the gene of interest, a process commonly referred to as gene "knockdown". Different types of inhibitory oligonucleotides are well-known in the art and include RNA molecules capable of inducing RNA interference such as microRNAs, small interfering RNAs or short hairpin RNAs as well as antisense oligonucleotides (ASOs).

An inhibitory oligonucleotide according to the invention is considered capable of selectively binding to the target sequence when it can at least partially hybridize to any of SEQ ID NO: 1, SEQ ID NO: 2, SEQ ID NO: 3, SEQ ID NO: 4, SEQ ID NO: 5, SEQ ID NO: 6 and SEQ ID NO: 7.

In the context of the invention, hybridization refers to a process wherein two single-stranded nucleic acid molecules anneal to each other via complementary Watson-Crick base pairing. In the case of DNA-RNA interactions, the nitrogenous base adenine present in the DNA strand thus pairs and forms hydrogen bonds with uracil in the opposing RNA strand, whereas adenine present in the RNA pairs with thymine in DNA. In the case of RNA-RNA interactions, the nitrogenous base adenine present in one RNA strand pairs and forms hydrogen bonds with uracil in the opposing strand. Regardless of whether the interactions occur between DNA and RNA or between two RNA molecules, cytosine pairs with guanine. Moreover, an inhibitory oligonucleotide is considered able to "target" ADAMTS7, when said oligonucleotide is able to selectively bind, i.e., to "hybridize" to a target mRNA encoding ADAMTS7 via complementary Watson-Crick base pairing as defined herein.

The part of an inhibitory oligonucleotide capable of selectively binding to any of SEQ ID NO: 1, SEQ ID NO: 2, SEQ ID NO: 3, SEQ ID NO: 4, SEQ ID NO: 5, SEQ ID NO: 6 and SEQ ID NO: 7 is herein referred to as "guide sequence". In some embodiments, the inhibitory oligonucleotide consists of said guide sequence, i.e., the inhibitory oligonucleotide hybridizes over its entire length to its respective target sequence within the ADAMTS7 mRNA. In other embodiments, the inhibitory oligonucleotide merely comprises the guide sequence. In such a scenario, the inhibitory oligonucleotide may thus comprise additional nucleic acids that are not part of the guide sequence and thus do not partake in the hybridization to the target sequence. Such additional nucleic acids may serve other functions instead, e.g., they may be involved in the assembly of higher-order structures, serve as linkers that connect the inhibitory oligonucleotide to other nucleic acid sequences, or they may be targeted for chemical modification to use them as anchors for attaching the inhibitory oligonucleotide, e.g., to a substrate of choice.

The guide sequence of the inhibitory oligonucleotide can, in some embodiments, have the same length as its target sequence. In other embodiments, the guide sequence of the inhibitory oligonucleotide of the invention may also be shorter than any of the target sequences according to SEQ ID NO: 1, SEQ ID NO: 2, SEQ ID NO: 3, SEQ ID NO: 4, SEQ ID NO: 5, SEQ ID NO: 6 or SEQ ID NO: 7. In still other embodiments, the guide sequence of the inhibitory oligonucleotide according to the invention may also bind a region of the target mRNA that overlaps with a target sequence according to any of SEQ ID NO: 1 to 7, i.e. the inhibitory oligonucleotide binds complementarily to a part of the target sequence according to SEQ ID NO: 1 to 7 as well as to nucleotides within the target mRNA which are located outside, e.g. adjacent to any of the target sequences of SEQ ID NO: 1 to 7. Thus, in the context of the invention, the guide sequence of an inhibitory oligonucleotide of the invention is considered complementary and thus capable of selectively binding to the target sequence if it consists of or comprises a guide sequence of complementary bases mirroring at least a part of the target sequence.

In a preferred embodiment, the inhibitory oligonucleotide consists of or comprises a guide sequence that is 100% complementary to any of SEQ ID NO: 1, SEQ ID NO: 2, SEQ ID NO: 3, SEQ ID NO: 4, SEQ ID NO: 5, SEQ ID NO: 6 and SEQ ID NO: 7., i.e., the guide sequence of the inhibitory oligonucleotide hybridizes over its entire length to the target mRNA sequence, wherein each base of the guide sequence hybridizes to a corresponding complementary base within the target mRNA sequence. Importantly, in the context of the invention, a guide sequence is also considered 100% complementary to any of SEQ ID NO: 1, SEQ ID NO: 2, SEQ ID NO: 3, SEQ ID NO: 4, SEQ ID NO: 5, SEQ ID NO: 6 or SEQ ID NO: 7 if it is shorter than the respective target sequence, as long as each individual nucleotide within the guide sequence binds complementarily to a corresponding nucleotide within the target sequence. In such a scenario, the inhibitory oligonucleotide molecule is highly specific to its target sequence and thus typically does not bind to other sequences within a given sample.

However, in some embodiments, it may be preferable that at least 1, e.g., at least 2, at least 3, at least 4, or at least 5 bases of the guide sequence of the inhibitory oligonucleotide have mismatches with their corresponding target mRNA sequence, i.e., the guide sequence does not bind with 100% complementarity to its corresponding target sequence. Accordingly, the guide sequence of the inhibitory oligonucleotide according to the invention may have a nucleic acid sequence that is merely 75-99%, e.g., 75%, 80%, 85%, 90%, 95% or 99% complementary to its target sequence. In such a scenario, the inhibitory oligonucleotide molecule still exhibits a very high specificity to its target sequence, while also allowing the recognition of variants thereof. This can be advantageous because the inhibitory oligonucleotide may then, e.g., be able to tolerate to some extent differences in the nucleic acid sequence of the ADAMTS7 gene among human populations.

In another embodiment, the guide sequence of the inhibitory oligonucleotide of the invention hybridizes to the target sequence with only a relatively short stretch of its sequence. For example, naturally occurring microRNAs (miRNAs) generally do not hybridize to a target RNA over their entire length. Rather, miRNAs only bind to their target mRNA with a short stretch of 6 to 8 nucleotides (nt) at their 5' end (the so-called seed sequence). Accordingly, the guide sequence of an inhibitory oligonucleotide according to the invention may already be considered selective for a target sequence within the meaning of the present invention when it is complementary to at least a seed region of at least 5 to 15, e.g., of at least 5, at least 6, at least 7, at least 8, at least 9, at least 10, at least 11, at least 12, at least 13, at least 14, or at least 15 nucleotides of its respective target.

The target sequences according to SEQ ID NO: 1, SEQ ID NO: 2, SEQ ID NO: 3, SEQ ID NO: 4, SEQ ID NO: 5, SEQ ID NO: 6 or SEQ ID NO: 7 within the *ADAMTS7* mRNA are characterized in that they are identically found in pigs and humans, i.e., they are conserved across pigs and humans. This is advantageous because pigs are often used as *in vivo* models in the preclinical research and pharmaceutical safety testing of new drugs as the anatomy, physiology, and biochemistry of pigs is relatively similar to that of humans. The target sequences of SEQ ID NO: 1, 2 and 3 are particularly preferred because they occur not only in pigs and humans but are also conserved in commonly used laboratory animal models including rabbits, rats and mice. SEQ ID NO:3 is even conserved in zebrafish. Given that SEQ ID NO: 1, 2 and 3 are conserved across rodent and non-rodent species, they are also more likely to be conserved across different human populations. The target sequences of SEQ ID NO: 1, 2 or 3 are all located in exon 8 of *ADAMTS7.*

The present inventors could show that inhibitory oligonucleotides that have been designed to selectively bind to the target sequences of SEQ ID NO: 1, SEQ ID NO: 2, SEQ ID NO: 3, SEQ ID NO: 4, SEQ ID NO: 5, SEQ ID NO: 6 or SEQ ID NO: 7 as defined herein exhibit a remarkable knockdown efficiency, as most of them were able to reduce the residual expression of ADAMTS7 in smooth muscle cells and human umbilical vein endothelial cells (HUVECs) to below 14% of the of ADAMTS7 expression levels in cells that were not exposed to these inhibitory oligonucleotides. Accordingly, the herein disclosed inhibitory oligonucleotide of the present invention capable of selectively binding to any of SEQ ID NO: 1, SEQ ID NO: 2, SEQ ID NO: 3, SEQ ID NO: 4, SEQ ID NO: 5, SEQ ID NO: 6 or SEQ ID NO: 7 is preferably further characterized in that it is capable of reducing the expression of ADAMTS7 in human SMCs to less than 14%, less than 13%, less than 12%, less than 11%, less than 10%, less than 9%, less than 8%, less than 7%, less than 6%, less than 5%, less than 4%, less than 3%, less than 2%, or less than 1 % of the ADAMTS7 expression levels in SMCs that were not exposed to the inhibitory oligonucleotide. In some embodiments, the inhibitory oligonucleotides according to the invention may even be capable of reducing ADAMTS7 expression in human SMCs to an undetectable level, i.e. the remaining expression level of ADAMTS7 is substantially 0%.

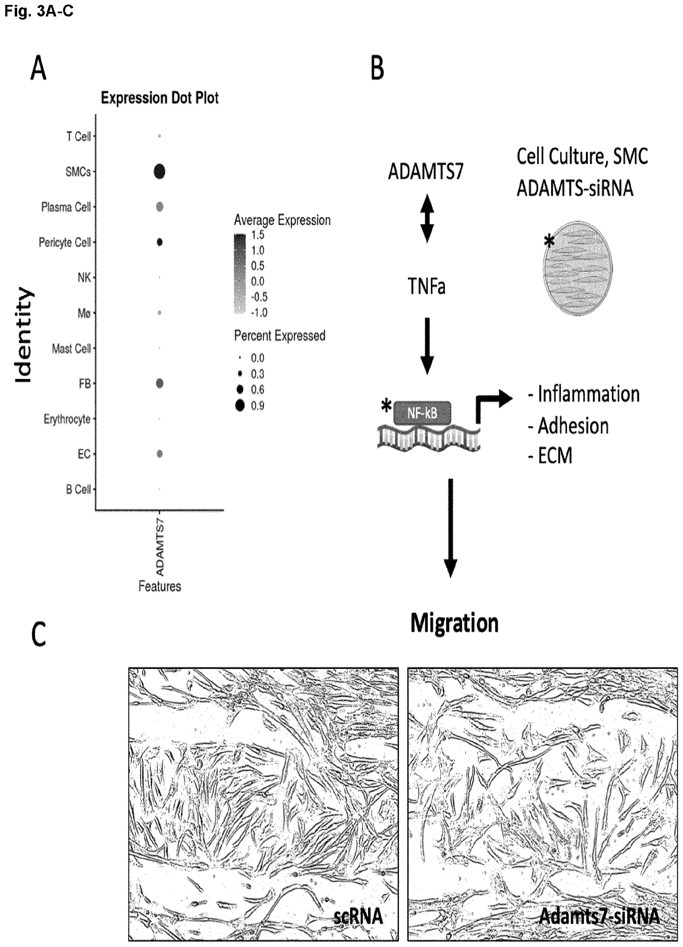

By conducting single cell RNAseq analysis of coronary arteries and using the interactive web application PlaqView (Ma et al., 2022) (www.plaqview.com), the present inventors found that ADAMTS7 is expressed predominantly in SMCs, (cf. Figure 3A), consistent with previous work showing that ADAMTS7 is expressed and localized to vascular smooth muscle in human atherosclerotic plaques (Pu et al., 2013). Thus, SMCs are the atherosclerosis-related cell type of choice for studying the regulation and function of ADAMTS7 in the context of CAD, particularly its role in expression of inflammatory ECM markers as well as ECM degradation markers. Furthermore, the effects of different known atherosclerosis-related stimuli on *ADAMTS7* gene regulation were tested, which revealed that *ADAMTS7* gene expression is stimulated by TNF-alpha (TNFα). The interaction between ADAMTS7 and TNFα triggers a sequence of events that significantly contribute to atherosclerosis progression, specifically through mechanisms involving smooth muscle cell migration or proliferation and phenotype switching. Thus, the present inventors established an *in vitro* assay to study the functional effects associated with the knockdown of *ADAMTS7* in human SMCs that relies on the measurement of SMC migration under pro-atherosclerotic conditions, i.e., in response to ADAMTS7 activation by TNFα.

Using this assay, it is thus possible to assess whether a given inhibitory oligonucleotide targeting ADAMTS7 mRNA is able to impact well-established atherosclerosis-related parameters such as SMC migration. As shown in the Examples below, the inventors found that several known and commercially available siRNAs capable of targeting human ADAMTS7 did not to exert any significant functional impact, i.e. did not noticeably reduce SMC migration. Accordingly, these inhibitory oligonucleotides were dismissed for use in therapeutic interventions against artherosclerosis and CAD.

The absence of functional effects of the known siRNAs in the above-described SMC migration assays can at least partially be attributed to the fact that the knockdown efficiencies of these oligonucleotides are comparably low, because, although the tested siRNAs can selectively bind to the ADAMTS7 mRNA, they still allowed for a comparably high residual expression levels of ADAMTS7 of above 14% in the treated cells. Of note, these known siRNAs do not selectively bind to any of the target sequences of SEQ ID NO: 1, 2, 3, 4, 5, 6, or 7.

In contrast, inhibitory oligonucleotides of the present invention that are able of complementarily binding to any of SEQ ID NO: 1, 2, 3, 4, 5, 6 or 7 as defined herein were found to reduce SMC migration and thus functionally impact atherosclerosis-related processes.

Inhibitory oligonucleotides that can selectively bind to SEQ ID NO: 1 have proven to be particularly effective. These oligonucleotides are not only characterized by a high knockdown efficiency of ADAMTS7 in SMCs and HUVECS, but also led to the strongest reduction in SMC migration. Moreover, inhibitory oligonucleotides that can selectively bind SEQ ID NO: 1 most strongly prevented the formation of tubule-like structures by HUVECS in established *in vitro* angiogenesis assays, suggesting that they can be effectively used to prevent or reduce angiogenesis and neovascularization. Neovascularization, i.e., the formation of new blood vessels, can occur within atherosclerotic plaques. Neovascularization in atherosclerosis can have detrimental effects leading to plaque instability and rupture. Further, neovascularization may promote inflammation and facilitate the recruitment of immune cells, further exacerbating plaque development and progression.

Thus, the inhibitory oligonucleotide of the invention preferably is an inhibitory oligonucleotide that is capable of selectively binding to the target nucleic acid sequence of SEQ ID NO: 1.

By employing bioinformatic analysis, the present inventors have specifically designed different inhibitory oligonucleotides that are capable of selectively binding to the different target sequences with the mRNA encoding *ADAMTS7.* These inhibitory oligonucleotides can comprise, consist of or correspond to at least subsequences of the nucleic acid sequences of any of SEQ ID NO: 8, 10, 12, 14, 16, 18, or 20.

SEQ ID NO: 8, 10, 12, 14, 16, 18 and 20 each have a length of 21 nt and are 100% complementary to the target sequences of SEQ ID NO: 1, 2, 3, 4, 5, 6, and 7, respectively, i.e., an inhibitory oligonucleotide consisting of or comprising SEQ ID NO: 8 selectively binds to a target sequence of SEQ ID NO: 1, an inhibitory oligonucleotide consisting of or comprising SEQ ID NO: 10 selectively binds to a target sequence of SEQ ID NO: 2, an inhibitory oligonucleotide consisting of or comprising SEQ ID NO: 12 selectively binds to a target sequence of SEQ ID NO: 3, an inhibitory oligonucleotide consisting of or comprising SEQ ID NO: 14 selectively binds to a target sequence of SEQ ID NO: 4, an inhibitory oligonucleotide consisting of or comprising SEQ ID NO: 16 selectively binds to a target sequence of SEQ ID NO: 5, an inhibitory oligonucleotide consisting of or comprising SEQ ID NO: 18 selectively binds to a target sequence of SEQ ID NO: 6, and an inhibitory oligonucleotide consisting of or comprising SEQ ID NO: 20 selectively binds to a target sequence of SEQ ID NO: 7.

The inhibitory oligonucleotides capable of selectively binding to any of SEQ ID NO: 1, SEQ ID NO: 2, SEQ ID NO: 3, SEQ ID NO: 4, SEQ ID NO: 5, SEQ ID NO: 6 or SEQ ID NO: 7 are either RNA molecules capable of inducing RNAi or antisense oligonucleotides (ASOs).

In a preferred embodiment, the inhibitory oligonucleotide is an RNA molecule capable of inducing a mechanism known as RNA interference (RNAi). RNAi is a natural and conserved molecular mechanism for post-transcriptional regulation of gene expression. In brief, RNAi involves the processing of double-stranded (ds) RNAs into shorter double stranded fragments of usually 20 to 25 base pairs (bp) inside a cell. Afterwards, the antisense strand of this dsRNA duplex, which is also referred to as guide strand, is incorporated into a multi-protein complex known as RNA-induced silencing complex (RISC). After association, this complex is recruited to a target mRNA molecule of interest via complementary base-pairing between the guide strand and a target sequence within said mRNA molecule, thereby blocking the translation of the mRNA transcript into a polypeptide. In instances where the antisense strand binds to its target sequence with near perfect complementarity, RNAi promotes the cleavage and irreversible degradation of the target RNA. Thus, RNAi can modulate both the stability and translational efficacy of mRNAs.

RNA molecules capable of inducing RNAi are referred herein also as "RNAi-triggers". RNAi triggers can be of natural origin due to transcription of endogenous genes and further processing of the resulting primary transcripts to yield the effective dsRNA duplexes. For instance, miRNAs are single-stranded, non-coding RNAs that are transcribed from endogenous miRNA-genes in the genome. It is estimated that hundreds of distinct and evolutionary conserved miRNAs modulate the expression of more than 60% of all human protein-coding genes within virtually every biomolecular pathway (Catalanotto et al., 2016). Physiologically, miRNAs are generated from longer endogenous precursor transcripts (pri-miRNA), that form hairpin structures and are processed through two distinct endonucleolytic cleavage steps in the nucleus (via the enzyme Drosha) and cytoplasm (via the enzyme Dicer) into short RNA duplexes with a length of of 20-23, e.g., 22 base pairs (bp) and a dinucleotide 3' overhang at both ends. Based on its thermodynamic properties defined by its sequence composition, the antisense strand of these duplexes is incorporated into RISC to guide the complex to a complementary target RNA.

The RNAi triggers of the present invention have been designed in silico. They can be introduced into cells using standard molecular biological techniques, e.g., via transfection of *in* vitro-generated RNAi triggers or via in situ expression of precursors of said RNAi triggers from, e.g., viral vectors.

Thus, if the inhibitory oligonucleotide of the invention capable of selectively binding to any of SEQ ID NO: 1, SEQ ID NO: 2, SEQ ID NO: 3, SEQ ID NO: 4, SEQ ID NO: 5, SEQ ID NO: 6 or SEQ ID NO: 7 is an RNA molecule capable of inducing RNAi, it preferably is a small interfering RNA (siRNA) or a short hairpin RNA (shRNA).

SiRNAs rely on a similar effector pathway as miRNAs but do not derive from endogenously transcribed hairpin structures. Instead, siRNAs can be either produced inside the cells from exogenous longer double stranded RNA fragments derived, e.g., from viral dsRNA, or they may be artificially designed and synthesized *in vitro* prior to their administration into cells.

Mature siRNAs typically have a length of 20 to 25 nt, preferably of 21 nt and, similar to miRNAs, rely on RNAi to prevent target RNAs from being translated into protein. However, whereas naturally occurring miRNAs typically silence tens to hundreds of genes by binding to the 3'UTR regions of the corresponding mRNAs guided by their 8 base pair "seed region" followed by repression of translation, synthetic siRNAs typically exhibit a higher specificity, often binding to their target sequences with up to 100% sequence complementarity, thereby inducing cleavage and degradation of their target mRNA via the RISC complex before translation.

Already shortly after the discovery of the RNAi pathway, scientist have recognized the therapeutic potential of synthetic siRNA-based drugs. The first siRNA therapeutic, Patisiran, was approved by the FDA in 2018 and many more are expected to follow. One challenge associated with siRNA-based drugs is site-specific delivery. This requires the use of suitable delivery systems as well as different chemical modifications that can stabilize the siRNA itself.

Short hairpin RNAs (shRNA) constitute an alternative to siRNA-based therapeutics. shRNAs are artificially designed single stranded RNA molecules that exhibit a characteristic hairpin turn. shRNAs typically have a length of 50-70 nt and form a stem-loop structure consisting of a 19 to 29 bp region of double-strand RNA (the stem) bridged by a region of predominantly single-stranded RNA (the loop) and a dinucleotide 3' overhang. Once they are introduced into a cell, they are processed similarly to miRNAs into short RNA duplexes that resemble siRNAs. One strand of this duplex is then incorporated into RISC to facilitate knock-down of a target gene. shRNAs can be encoded by an expression vector, such as a viral vector, which allows expression and processing of the shRNA construct inside a target cell. Optionally, they can also be produced by *in vitro* transcription of a linearized plasmid-based expression construct or via chemical oligonucleotide synthesis, e.g., by using nucleoside phosphoramidites as building blocks. Additionally, shRNAs may be expressed from expression plasmids, such as CpG-free expression vector plasmids.

shRNAs are normally transcribed by RNA polymerase III. However, the expression of exogenous shRNAs in cells can saturate the endogenous miRNA machinery, causing toxic effects to the cell. Moreover, imprecise processing of the stem loop structures of shRNAs may result in aberrant guide- and passenger-strand mediated off-target effects. To overcome these problems, shRNAs may be provided as a "shRNAmiRs", i.e., the shRNA is embedded within the backbone of a natural miRNA. The skilled person is aware of suitable miRNA backbones that can be used for the provision of shRNAs to cells, which may include e.g. the backbone of miR30a, miR223, miR144 or miR33 and they may be generated as described in e.g., Adams et al., 2017, which is hereby incorporated by reference. Such a shRNAmiR may also be referred to as an artificial miRNA or miRNA mimic. shRNAmiRs can be generated from Polymerase II promoters, enabling constitutive, tissue specific or inducible expression (Adams et al., 2017), as well as physiological expression levels.

The RNA molecule capable of inducing RNAi according to the invention may comprise a nucleic acid sequence having at least 90%, preferably at least 95%, and most preferably 100% sequence identity to any of SEQ ID NO: 8, SEQ ID NO: 10, SEQ ID NO: 12, SEQ ID NO: 14, SEQ ID NO: 16, SEQ ID NO: 18 or SEQ ID NO: 20, preferably to any of SEQ ID NO: 8, SEQ ID NO: 10, or SEQ ID NO: 12. The RNA molecule capable of inducing RNAi according to the invention may also consist of a nucleotide sequence having at least 90%, preferably at least 95%, and most preferably 100% sequence identity to any of SEQ ID NO: 8, SEQ ID NO: 10, SEQ ID NO: 12, SEQ ID NO: 14, SEQ ID NO: 16, SEQ ID NO: 18 or SEQ ID NO: 20, preferably to any of SEQ ID NO: 8, SEQ ID NO: 10, or SEQ ID NO: 12. Most preferably, the RNA molecule capable of inducing RNAi according to the invention may comprise or consist of a nucleic acid sequence having at least 90%, 95% or 100% sequence identity to SEQ ID NO: 8.

The skilled person will be aware that neither siRNAs nor shRNAs must be fully complementary to their target sequences to effectively suppress protein synthesis, i.e., he or she will know that some mismatches are admissible. Thus, if the herein disclosed siRNA antisense strand or the guide sequence of the shRNA comprises one or two substitutions compared to any of SEQ ID NO: 8, 12, 14, 16, 18 or 20, it binds to its respective target sequence with imperfect complementarity but can nevertheless be a highly selective and potent RNAi trigger that specifically and effectively induces cleavage of the target RNA or inhibits its translation into protein.

Thus, the RNA molecule capable of inducing RNAi according to the invention may comprise or consist of a guide sequence that comprises up to 2, i.e., 0, 1 or 2 nucleotide substitutions compared to any of SEQ ID NO: 8, 10, 12, 14, 16, 18 or 20. The RNA molecule capable of inducing RNAi according to the invention may also comprise or consist of a guide sequence that comprises up to 2, i.e., 0, 1 or 2 nucleotide deletions compared to any of SEQ ID NO: 8, 10, 12, 14, 16, 18 or 20, in which case preferably only nucleotides located at the 3' or 5' ends of said guide sequence are deleted and not nucleotides within said guide sequence to avoid frameshifts within the guide sequence that prevent complementary binding of the inhibitory oligonucleotide to the target sequence.

However, in preferred embodiments, the nucleotides corresponding to positions 2-8 of SEQ ID NO: 8 to 14 are not substituted in the herein disclosed RNAi triggers, e.g., the siRNAs or shRNAs, as these positions form the so-called "seed" region critical for the specificity of an RNAi trigger. Moreover, multiple studies (e.g., Du et al., 2005; Ahmed and Raghava, 2011) suggest that mismatches affecting positions 4 -12 in siRNAs can reduce RNAi efficacy, whereas mismatches at positions 1, 2, 3, 18 and 19, in particular at positions 1 and 19, appear to affect RNAi efficacy only weakly or not at all (Ahmed and Raghava, 2011).

In view of this, if the RNA molecule of the invention capable of inducing RNAi comprises or consists of a nucleic acid sequence having 90% or 95% sequence identity to any of SEQ ID NO: 8, 10, 12, 14, 16, 18 or 20, it preferably does not comprise a substitution at a position that correspond to positions 2-12 of SEQ ID NO: 8, 10, 12, 14 16, 18 or 20. It may, e.g., comprise a substitution at a position that corresponds to position 1 and/or position 19 of SEQ ID NO: 8, 10, 12, 14 16, 18 or 20. An RNA molecule of the invention capable of inducing RNAi may also comprise or consist of a nucleic acid sequence that is shorter than the sequences of SEQ ID NO: 8, 10, 12, 14 16, 18 or 20, e.g., it may comprise or consist only of a continuous subsequence spanning 19 or 20 nucleotides, e.g., the first 19 or 20 nt, of any of SEQ ID NO: 8, 10, 12, 14, 16, 18 or 20.

In some embodiments, the inhibitory oligonucleotide of the invention is an siRNA. An siRNA is typically provided as an RNA duplex of 20 to 27 base pairs (bp) consisting of a sense and a complementary antisense strand with characteristic 1 or 2 nt single-stranded overhangs on both sites. The antisense strand of the siRNA duplex serves as the guide strand, which is incorporated into RISC to target the complex to its respective target sequence within the *ADAMTS7* mRNA to induce RNAi-mediated gene knock-down. The antisense strand of the siRNA duplex may thus comprise a nucleic acid sequence having at least 90%, preferably at least 95%, more preferably 100% sequence identity to any of SEQ ID NO: 8, 10, 12, 14, 16, 18 or 20, preferably to SEQ ID NO: 8, 10 or 12, most preferably to SEQ ID NO: 8. Alternatively, the antisense strand of the siRNA duplex may also consist of a nucleic acid sequence having at least 90%, preferably at least 95%, more preferably 100% sequence identity to any of SEQ ID NO: 8, 10, 12, 14, 16, 18 or 20, preferably to SEQ ID NO: 8, 10 or 12, most preferably to SEQ ID NO: 8. A siRNA duplex comprising an antisense strand of SEQ ID NO: 8 may comprise a sense strand of SEQ ID NO: 9, a siRNA duplex comprising an antisense strand of SEQ ID NO: 10 may comprise a sense strand of SEQ ID NO: 11, a siRNA duplex comprising an antisense strand of SEQ ID NO: 12 may comprise a sense strand of SEQ ID NO: 13, a siRNA duplex comprising an antisense strand of SEQ ID NO: 14 may comprise a sense strand of SEQ ID NO: 15, a siRNA duplex comprising an antisense strand of SEQ ID NO: 16 may comprise a sense strand of SEQ ID NO: 17, a siRNA duplex comprising an antisense strand of SEQ ID NO: 18 may comprise a sense strand of SEQ ID NO: 19, and a siRNA duplex comprising an antisense strand of SEQ ID NO: 20 may comprise a sense strand of SEQ ID NO: 21.

In other embodiments, the inhibitory oligonucleotide may also be only one strand of an siRNA duplex, i.e. it may only be the antisense strand comprising or consisting of a nucleic acid sequence having at least 90%, preferably at least 95%, more preferably 100% sequence identity to any of SEQ ID NO: 8, 10, 12, 14, 16, 18 or 20. In such a scenario, the inhibitory oligonucleotide may also consist of a nucleic acid sequence having at least 90%, preferably at least 95%, more preferably 100% sequence identity any of SEQ ID NO: 8, 10, 12, 14, 16, 18 or 20.

In alternative embodiments, the RNA molecule capable of inducing RNAi may be an shRNA, whereas, optionally, a nucleic acid sequence (guide sequence) having at least 90%, preferably at least 95%, most preferably 100% sequence identity to any of SEQ ID NO: 8, 10, 12, 14, 16, 18 or 20 forms a part of the stem of said shRNA. Accordingly, when the RNA molecule is an shRNA, it necessarily comprises additional nucleotides besides the guide sequence having, e.g., at least 90%, preferably at least 95%, most preferably 100% sequence identity to any of SEQ ID NO: 8, 10, 12, 14, 16, 18 or 20 to facilitate proper folding of the RNA molecule into the stem loop structure of an shRNA.

Thus, the inhibitory oligonucleotide capable of selectively binding to any of SEQ ID NO: 1 , SEQ ID NO: 2, SEQ ID NO: 3, SEQ ID NO: 4, SEQ ID NO: 5, SEQ ID NO: 6, or SEQ ID NO: 7 may be an shRNA comprising a guide sequence having at least 90%, at least 95%, or 100% sequence identity to any of SEQ ID NO: 8, 10, 14, 16, 18 or 20. Preferably, the shRNA comprising a guide sequence having at least 90%, at least 95%, or 100% sequence identity to any of SEQ ID NO: 8, 10 or 12, most preferably at least 90%, at least 95%, or 100% sequence identity to SEQ ID NO: 8.

Particular preferred shRNAs of the invention comprise a guide sequence having at least 90%, preferably at least 95%, most preferably 100% sequence identity to any of SEQ ID NO: 22-28. A guide sequence of SEQ ID NO: 22 is able of selectively binding to a target sequence of SEQ ID NO: 1, a guide sequence of SEQ ID NO: 23 is able of selectively binding to a target sequence of SEQ ID NO: 2, guide sequence of SEQ ID NO: 24 is able of selectively binding to a target sequence of SEQ ID NO: 3, a guide sequence of SEQ ID NO: 25 is able of selectively binding to a target sequence of SEQ ID NO: 4, a guide sequence of SEQ ID NO: 26 is able of selectively binding to a target sequence of SEQ ID NO: 5, a guide sequence of SEQ ID NO: 27 is able of selectively binding to a target sequence of SEQ ID NO: 6 and a guide sequence of SEQ ID NO: 28 is able of selectively binding to a target sequence of SEQ ID NO: 7. In particular preferred embodiments, the shRNA of the invention comprises a guide sequence having at least 90%, preferably at least 95%, most preferably 100% sequence identity to any of SEQ ID NO: 22-24. Most preferably, the shRNA of the invention comprises a guide sequence having at least 90%, preferably at least 95%, most preferably 100% sequence identity to SEQ ID NO: 22.

Preferably, the shRNA is embedded into a miRNA backbone, also referred to as miRNA framework, as described herein, e.g., the framework of miR30a, miR223, miR144 or miR33. Accordingly, the RNA molecule may also be an shRNAmiR.

The RNA molecule capable of inducing RNAi of the invention may comprise one or more chemical modifications that have a stabilizing effect, in particular, in case the RNA molecule is an siRNA. For instance, the RNA molecule may comprise a modification in its ribose sugar backbone, e.g., a 2'-O modification such as 2'-OMe, 2'-F, 2'-O-methoxyethyl (2'-MOE) or 2'-O-guanidinopropyl (2'-O-GP). In particular nucleotides 2-5 of the siRNA antisense strands according to the invention may preferably comprise a 2'-OMe modification to further reduce the risk of possible off-target effects.

Additional modifications that may be incorporated in the RNA molecule of the invention may include, e.g., 5-fluoro-2'-deoxyuridine and 2'-O-methyl phospshorodithioate moieties.

The aforementioned modifications as well as others that may be used in the herein disclosed RNA molecules are well known to the skilled person and are summarized e.g., in Selvam et al., 2017. Modifications may also be added to improve siRNA delivery as described in more detail below. For instance, one or more nucleotides in the passenger RNA strand of the siRNA duplex may be conjugated to a suitable lipophilic moiety or anchor such as, e.g., palmityl-(also referred to as 2'-O-hexadecyl or C16) or cholesterol-residues. Palmityl modifications of siRNAs are known to the skilled person due to, e.g. Brown et al., 2022.

In another embodiment of the invention, the inhibitory oligonucleotide capable of selectively binding to a target nucleic acid sequence according to any of SEQ ID NO: 1 to 7 is not an RNA molecule capable of inducing RNAi but an antisense oligonucleotide (ASO). ASOs are artificial single-stranded oligonucleotides of about 12-25 nt, more typically of 15-22 nt length that target RNAs via complementary base pairing. They are usually made of DNA that optionally comprises various chemical modifications to increase their efficacy, enzymatic stability and decrease their immunogenicity and off-target toxicity. A specific form of ASOs are so-called gapmers, chimeric oligonucleotides formed of a central region made of DNA nucleotides flanked by stretches of chemically modified ribonucleotides such as locked nucleic acids (LNAs).

ASOs exert their effects by two main mechanisms. On the one hand, ASOs can hybridize to their target RNAs to form DNA-RNA hybrids, which are recognized by RNase H, an endonuclease that triggers cleavage of the RNA strand of the DNA-RNA duplex. Gapmers in particular are specifically designed to catalyze RNase H-dependent degradation of complementary RNA targets. On the other hand, binding of ASOs to functional cis-acting elements within the target RNA may sterically block access of the cellular machinery to the RNA to, e.g., prevent translation or interfere with the splicing of the RNA.

The therapeutic use of ASOs is commonly referred to as antisense therapy.

In some embodiments, the ASO according to the invention comprises or consists of a nucleic acid sequence that corresponds to a subsequence of at least 6 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20, preferably of SEQ ID NO: 8, SEQ ID NO: 10, or SEQ ID NO: 12, most preferably of SEQ ID NO: 8.

SEQ ID NO: 8, 10, 12, 14, 16, 18 and 20 relate to ribonucleic acid sequences. However, it is to be understood that a nucleic acid sequence that corresponds to SEQ ID NO: 8, 10, 12, 14, 16, 18 or 20 or a subsequence thereof may be a deoxyribonucleic acid sequence or ribonucleic acid sequence. It may even comprise both deoxyribonucleotides and ribonucleotides and/or modified nucleotides, e.g., in case the ASO is a chimeric gapmer, as described in more detail below.

The subsequence of at least 6 nucleotides may correspond to any continuous stretch of at least 6 nt length present in any of SEQ ID NO: 8, 10, 12, 14, 16, 18 or 20. For instance, the ASO may comprise a nucleic acid sequence that corresponds to at least the first or final 6 nucleotides of any of SEQ ID NO: 8, 10, 12, 14, 16, 18 or 20. Preferably, the ASO comprises a nucleic acid sequence that corresponds to a subsequence of any of SEQ ID NO: 8, 10, 12, 14, 16, 18 or 20 that is longer than 6 nt, e.g., it may comprise a nucleic acid sequence that corresponds to a continuous subsequence spanning at least 10, e.g., at least 11, 12, 13, 14, 15, 16, 17, 18, 19, or 20 nt of any of SEQ ID NO: 8, 10, 12, 14, 16, 18 or 20. Preferably, the ASO comprises a nucleic acid sequence that corresponds to a subsequence of at least 12, at least 14, or at least 16 nucleotides of any of SEQ ID NO: 8, 10, 12, 14, 16, 18 or 20. In some embodiments, the ASO may also comprise a nucleic acid sequence that corresponds to the complete sequence of any of SEQ ID NO: 8, 10, 12, 14, 16, 18 or 20 or consist thereof. Preferably, the ASO comprises a nucleic acid sequence that corresponds to a subsequence of at least 10 or more continuous nucleotides of any of SEQ ID NO: 8, 10 or 12, more preferably of SEQ ID NO: 8. If the candidate oligonucleotide is an ASO, the guide sequence is preferably fully complementary to the respective target sequence. Particular preferred ASOs of the invention have a nucleic acid sequence of any of SEQ ID NO: 29, 30, 31, 32, 33, 34 or 35. Most preferably, the ASO of the invention has a nucleic acid sequence of SEQ ID NO: 29.

The ASO of the invention may be a DNA ASO.

The ASOs of the invention may comprise a variety of chemical modifications to improve their pharmacokinetic properties such as stability, specificity and membrane permeability, and minimize their cytotoxicity. For instance, the non-bridging oxygen atoms in the phosphate group of the nucleotides forming the ASO may be replaced by phosphorothioate (PS), resulting in the formation of PS bonds that are resistant to nuclease-based degradation. Moreover, the 2' position of the ribose may be modified with an alkyl moiety, such as a methyl (2'-OMe) or a methoxyethyl (2'-MOE) group. The ASO may also be a locked nucleic acid (LNA), a peptide nucleic acid (PNA) or a phosphorodiamidate morpholino oligomer (PMO). LNAs contain a constrained ribose ring having a O2'-C4'-methylene linkage. PNAs have a peptide-like N-(2-aminoethyl) glycine linkage to replace the ribose-phosphate DNA backbone. PMOs contain a backbone of morpholine rings connected by phosphorodiamidate linkages. Such modified ASOs are characterized by a high target affinity, improved pharmacokinetic profiles and nuclease resistance (Tarn et al, 2021).

In further embodiments, the herein disclosed ASOs may be gapmers, i.e., chimeric ASOs that consist of a central region of deoxyribonucleotides flanked by a stretch of ribonucleotides in which the ribose ring is modified with 2'-OMe, 2'-MOE or LNA. Gapmers can induce RNase H-mediated cleavage of the target RNA with a relatively greater binding affinity and specificity than conventional ASOs (Tarn et al, 2021).

Additional chemical modifications that can be introduced into ASOs are described in the state of the art, e.g., in Tarn et al., 2021. Based on the herein disclosed information and the state of the art, the skilled person will have no difficulties in designing functional ASOs comprising the herein disclosed nucleic acid sequences or subsequences thereof.

As for siRNAs, the herein disclosed ASOs may further comprise modifications to improve ASO delivery. However, since a PS backbone assists the membrane translocation of ASOs, additional means of delivery may be dispensable for PS-ASOs. Nevertheless, the herein disclosed ASOs may as well be conjugated to suitable lipophilic moieties or anchors such as, e.g., palmityl- (also referred to as 2'-O-hexadecyl or C16) or cholesterol-residues as described above. ASOs may also be conjugated to cell-penetrating peptides (CPPs) for enhanced drug delivery, such as the polycationic HIV-1 Tat peptide, the hydrophobic residue-containing peptide and artificial poly-arginine peptides. ASOs made of PMO have also been conjugated in the past to a synthetic octa-guanidine dendrimer. Such conjugates are referred to as vivo-PMOs (Tarn et al., 2021).

The present invention further relates to a composition providing at least one of the inhibitory oligonucleotides as disclosed herein, i.e., the composition provides at least a first inhibitory oligonucleotide capable of selectively binding to any of SEQ ID NO: 1, 2, 3, 4, 5, 6 or 7, preferably, SEQ ID NO: 1. It is to be understood that the terms "one of the inhibitory oligonucleotides" or "a first inhibitory oligonucleotide" does not mean that the composition provides only a single molecule of said inhibitory oligonucleotide. Instead, these terms should be understood to mean that the composition provides a plurality of at least one of the inhibitory oligonucleotides disclosed herein, e.g., a plurality of siRNAs comprising an antisense strand of SEQ ID NO: 8.

The first inhibitory oligonucleotide may be any of the inhibitory oligonucleotides disclosed in the context of the invention, e.g., it may be an RNA molecule capable of inducing RNAi, such as an siRNA or an shRNA, that either comprises or consists of a nucleic acid having at least 90%, preferably at least 95%, most preferably 100% sequence identity to any of SEQ ID NO: 8, 10, 12, 14, 16, 18 or 20 or it may be an ASO that comprises or consists of a nucleic acid sequence that corresponds to a subsequence of at least 6 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20, e.g., an ASO of any of SEQ ID NO: 29, 30, 31, 32, 33, 34 or 35.

Preferably, however, the composition provides combinations of more than one of the inhibitory oligonucleotides disclosed herein, e.g., it may provide at least a second, a third or even a fourth inhibitory oligonucleotide capable of selectively binding to any of SEQ ID NO: 1, 2, 3, 4, 5, 6 or 7. It may even provide a fifth, a sixth or seventh inhibitory oligonucleotide as disclosed herein. Again, any reference to a second, third, fourth, etc. inhibitory oligonucleotide is intended to mean that the composition provides a plurality of a first inhibitory oligonucleotide, a plurality of a second inhibitory oligonucleotide, a plurality of a third inhibitory oligonucleotide, a plurality of a fourth inhibitory oligonucleotide, etc.

If the composition provides combinations of several of the herein disclosed inhibitory oligonucleotides, the inhibitory oligonucleotides provided by the composition can bind to different target sequences and/or be of different types, e.g., siRNAs, shRNAs orASOs. Typically, they bind to different target sequences and are of the same type.

For instance, in a possible embodiment of the invention, the composition could provide, e.g., a first inhibitory oligonucleotide capable of selectively binding to a target sequence of SEQ ID NO: 1, a second inhibitory oligonucleotide capable of selectively binding to a target sequence of SEQ ID NO: 2, a third inhibitory oligonucleotide capable of selectively binding to a target sequence of SEQ ID NO: 3, a fourth inhibitory oligonucleotide capable of selectively binding to a target sequence of SEQ ID NO: 4, a fifth inhibitory oligonucleotide capable of selectively binding to a target sequence of SEQ ID NO: 5, a sixth inhibitory oligonucleotide capable of selectively binding to a target sequence of SEQ ID NO: 6, and a sevenths inhibitory oligonucleotide capable of selectively binding to a target sequence of SEQ ID NO: 7.

In addition or alternatively, a composition providing more than one of the inhibitory oligonucleotides disclosed herein may provide, e.g., as a first inhibitory oligonucleotide, an siRNA capable of selectively binding to a target sequence of SEQ ID NO: 1, as a second inhibitory oligonucleotide, an shRNA capable of selectively binding to a target sequence of SEQ ID NO: 1 and, as a third inhibitory oligonucleotide, an ASO capable of selectively binding to a target sequence of SEQ ID NO:1.

Thus, at least in principle, the composition may comprise any conceivable combination of all the different inhibitory oligonucleotides disclosed herein. However, preferably, inhibitory oligonucleotides that are capable of selectively targeting SEQ ID NO: 1 and 2 are not combined in a single composition as these two target sequences overlap and thus share a high degree of sequence similarity.

Preferred compositions according to the invention may provide, e.g., a combination of inhibitory oligonucleotides capable of selectively binding to SEQ ID NO: 1 and 3, a combination of inhibitory oligonucleotides capable of selectively binding to SEQ ID NO: 2 and 3, a combination of inhibitory oligonucleotides capable of selectively binding to SEQ ID NO: 1, 3, 4 and 5 or a combination of inhibitory oligonucleotides capable of selectively binding SEQ ID NO: 2, 3, 4 and 5.

For instance, compositions according to the invention may provide a combination of an siRNA comprising an antisense strand of SEQ ID NO: 8 and an siRNA comprising an antisense strand of SEQ ID NO: 12, a combination of an siRNA comprising an antisense strand of SEQ ID NO: 10 and an siRNA comprising an antisense strand of SEQ ID NO: 12, a combination of an siRNA comprising an antisense strand of SEQ ID NO: 8, an siRNA comprising an antisense strand of SEQ ID NO: 12, an siRNA comprising an antisense strand of SEQ ID NO: 14 and an siRNA comprising an antisense strand of SEQ ID NO: 16 or a combination of an siRNA comprising an antisense strand of SEQ ID NO: 10, an siRNA comprising an antisense strand of SEQ ID NO: 12, an siRNA comprising an antisense strand of SEQ ID NO: 14 and an siRNA comprising an antisense strand of SEQ ID NO: 16.

Alternatively, compositions according to the invention may provide a combination of an shRNA comprising SEQ ID NO: 22 and an shRNA comprising SEQ ID NO: 24, a combination of an shRNA comprising SEQ ID NO: 23 and an shRNA comprising SEQ ID NO: 24, a combination of an shRNA comprising SEQ ID NO: 22, an shRNA comprising SEQ ID NO: 24, an shRNA comprising SEQ ID NO: 25 and an shRNA comprising SEQ ID NO: 26 or combination of an shRNA comprising SEQ ID NO: 23, an shRNA comprising SEQ ID NO: 24, an shRNA comprising SEQ ID NO: 25 and an shRNA comprising SEQ ID NO: 26.

The co-provision of combinations of several of the inhibitory oligonucleotides disclosed herein within a single composition is advantageous as it may increase the efficacy of knockdown while reducing the risk of toxicity and off-target effects. The latter results from the fact that due to the combination of different inhibitory oligonucleotides, each of the different oligonucleotides can be provided at lower concentrations.

In the context of the invention, the phrase "the composition provides" is to be understood to mean either that the composition comprises one or more of the inhibitory oligonucleotides of the invention, or, in particular when the inhibitory oligonucleotide is an shRNA, that the composition comprises a delivery vehicle configured to express the shRNA in situ from a nucleic acid encoding the shRNA.

In the first scenario, according to which the composition comprises one or more of the inhibitory oligonucleotides according to the invention, the inhibitory oligonucleotides according to the invention are first prepared *in vitro* before being incorporated into a composition. The *in vitro* preparation of the different types of inhibitory oligonucleotides is carried out according to standard procedures known to the skilled person from the prior art. For example, oligonucleotides can be generated by chemical synthesis, which is typically done by commercial companies on a customized basis. Alternative methods for generating RNA molecules capable of inducing RNAi are well known to the skilled person and include, e.g., in vitro-transcription or in vivo transcription from DNA templates introduced into cells.

If the composition comprises one or more in vitro prepared inhibitory oligonucleotides of the invention, the composition preferably further comprises a suitable delivery vehicle that encompasses said inhibitory oligonucleotides or is associated with said inhibitory oligonucleotides.

A person skilled in the art is aware of various types of vehicles for delivering in vitro generated inhibitory oligonucleotides such as ASOs, siRNAs or *in* vitro-transcribed shRNAs and how they can be selectively targeted to cells and tissues of interest.

In a preferred embodiment, the delivery vehicle may e.g., comprise a lipid membrane that encapsulates the inhibitory oligonucleotide. For example, the vehicle may be a liposomal or an exosomal carrier comprising the inhibitory oligonucleotide as a cargo and, optionally, expressing a targeting moiety on its surface.

In a particularly preferred embodiment, the composition comprises as a vehicle a lipid nanoparticle comprising one or more of the inhibitory oligonucleotides of the invention. Lipid nanoparticles for use as drug delivery vehicles for, e.g., siRNAs were first approved in 2018. A lipid nanoparticle is typically spherical with an average diameter between 10 and 1000 nanometers. Solid lipid nanoparticles possess a solid lipid core matrix that can solubilize lipophilic molecules. The lipid core is typically stabilized by surfactants (emulsifiers). The emulsifier used depends on administration routes and is more limited for parenteral administrations. The lipids used for generating a lipid nanoparticle may include triglycerides (e.g. tristearin), diglycerides (e.g. glycerol bahenate), monoglycerides (e.g. glycerol monostearate), fatty acids (e.g. stearic acid), steroids (e.g. cholesterol), and waxes (e.g. cetyl palmitate) (https://en.wikipedia.org/wiki/Solid_lipid nanoparticle). Typical next generation lipid nanoparticle compositions also comprise varying composition of structural lipids (e.g. MC3), cholesterol or beta-sitosterol, PEG (e.g., DSPG-PEG2000 or DMG-PEG2000) and ionisable or cationic lipids (e.g., DOPE or DOTAP).

The vehicle may also be a bacterial minicell or an extracellular vesicle. These types of carriers have the ability to entrap both hydrophilic therapeutic agents within their central aqueous core or lipophilic drugs within their bilayer compartment.

Use of liposomal, exosomal, or minicell delivery vehicles and nanoparticles has the further benefit of allowing for fusion of the vehicle with the lipid membrane of the cell of interest e.g., during endocytosis to allow for the release of inhibitory oligonucleotides into the cytoplasm of the cell.

In another embodiment, the vehicle may be a polymeric micro- or nanoparticle. The micro- or nanoparticle may be formed of any physiologically acceptable polymeric material known in the art, e.g., it may comprise a biopolymer such as polysaccharide selected from the group comprising cellulose, alginate or chitosan.

The vehicle may also be a micro- or nanoparticle formed by a cationic polymer that is associated with one or more of the inhibitory oligonucleotides disclosed herein. Suitable cationic polymers comprise, e.g., polyethylenimine (PEI) or DEAE-dextran, or branched polymeric molecules, e.g., hyperbranched polymers such as hyperbranched polyglycerol polymers or dendrimers. The association between the cationic polymer and the one or more inhibitory oligonucleotides of the invention can be covalent or non-covalent, but typically is non-covalent. The cationic polymer, e.g., polyethyleneimine (PEI) is capable of complexing with nucleic acids via electrostatic interactions between its cationic groups and the negatively charged nucleic acids, thereby forming so called polyplexes. These polyplexes are characterized by high transfection efficiency due to the high buffering capacity of PEI, which facilitates endo-somal escape of the gene payload (Schwarz and Merkel et al., 2017). The cationic polymers, e.g., the PEI vectors, can be functionalized with a wide variety of polymers including oligosaccharides, polyethylene glycol) (PEG), poly(ε-caprolactone), and others. They may further carry, e.g., targeting moieties or comprise chemical modifications such as ester or disulfide bonds to yield degradable PEI analogs that exhibit reduced cytotoxicity.

The *in* vitro-generated inhibitory oligonucleotide may also not be incorporated into a delivery vehicle as described herein but may alternatively be labelled with a suitable lipid anchor to improve the delivery of the inhibitory oligonucleotide, as already described herein. Thus, in some embodiments, the composition comprises the herein disclosed inhibitory oligonucleotides of the invention linked to a lipophilic moiety. Exemplary lipophilic moieties for delivering RNAi triggers such as siRNAs and shRNAs or for delivery of ASOs include lipid residues such as palmityl- (also referred to as 2'-O-hexadecyl or C16) or cholesterol-residues as described, e.g., in Raouane et al., 2012 or Brown et al., 2022.

The inhibitory oligonucleotide may also be complexed with cationic lipids such as Lipofectamine 2000.

The herein described vehicles for delivering in vitro-produced inhibitory oligonucleotides can release the inhibitory oligonucleotides at once, e.g., after the vehicle has reached its targeted destination inside the subject. Alternatively, the vehicle may be configured in a fashion to allow for a sustained release of the *in vitro* produced inhibitory oligonucleotides in a desired period of time. A person skilled in the art will be able to choose a suitable vehicle from the art depending on the type of inhibitory oligonucleotide used, the route of administration, and the respective target cell to which the inhibitory oligonucleotide is to be delivered. In addition, based on the known state of the art, the skilled person will be able to further modify the vehicle for optimized targeted delivery and release of the oligonucleotide.

When the vehicle is, e.g., a lipid nanoparticle, a cationic polymer such as, e.g., a PEI vector or a lipophilic moiety such as 2'-O-hexadecyl, the inhibitory oligonucleotide is preferably provided in the form of any of the herein disclosed siRNAs or ASOs. The inhibitory oligonucleotide may however also be provided as an *in vitro* produced shRNA or shRNAmiR. In some embodiments, the delivery vehicle, e.g., the lipid nanoparticle or the cationic polymer, comprises only inhibitory oligonucleotides of the same type, e.g., the lipid nanoparticle comprises only RNA molecules capable of inducing RNAi or only ASOs. The herein disclosed delivery vehicles may further comprise mixtures of inhibitory oligonucleotides comprising or consisting of different nucleic acid sequences, e.g. RNA molecules comprising or consisting of SEQ ID NO: 8 and RNA molecules comprising or consisting of SEQ ID NO: 12 or RNA molecules comprising or consisting of SEQ ID NO: 10 and RNA molecules comprising or consisting of SEQ ID NO: 12. Alternatively, the delivery vehicle may also comprise a combination of the different ASOs disclosed herein. The delivery vehicles may also comprise a mixture of any of the herein disclosed RNAi triggers and ASOs. In such a scenario, the RNA molecules and ASOs present in the delivery vehicle may bind to the same target sequence or to different ones.

The composition may also comprise several delivery vehicles as described herein, in which case each delivery vehicle comprises an inhibitory oligonucleotide comprising or consisting of a different nucleic acid sequence disclosed herein. For instance, the therapeutic may comprise a first delivery vehicle, comprising an RNA molecule comprising or consisting of a nucleic acid sequence of SEQ ID NO: 8, and a second delivery vehicle comprising an RNA molecule comprising or consisting of a nucleic acid sequence of SEQ ID NO: 12. The composition may also comprise several delivery vehicles, wherein some delivery vehicles within the composition comprise any of the herein disclosed RNA molecules and some delivery vehicles within the composition comprise any of the herein disclosed ASOs. In such a scenario, the RNA molecules and ASOs used in the composition may either bind to the same target sequence or to different ones, preferably to different ones. The composition may also comprise several different oligonucleotide molecules of the invention linked to a liphophilic moiety such as 2'-O-hexadecyl (C16). For instance, the therapeutic may comprise a mixture of RNA molecules comprising or consisting of SEQ ID NO: 8 conjugated to 2'-O-hexadecyl and RNA molecules comprising or consisting of SEQ ID NO: 12 conjugated to 2'-O-hexadecyl.

When the composition comprises only a single inhibitory oligonucleotide, e.g., only an inhibitory oligonucleotide capable of binding to a target sequence of SEQ ID NO: 1 such as an siRNA comprising an antisense strand of SEQ ID NO: 8, the inhibitory oligonucleotide may be present in the composition at a concentration of, e.g., about 5-15 nM, e.g., 6-14 nM, 7-13 nM, 8-12 nM, 9-11 nM or 10 nM. For instance, the present inventors found that an siRNA comprising an antisense strand consisting of any of SEQ ID NO: 8, 10 or 12 exhibited a robust knockdown efficiency and reduced the ADAMTS7 expression in SMCs to below 10% when being used at a concentration of 10 nM. However, when the composition comprises more than one inhibitory oligonucleotide, e.g. two of the herein disclosed inhibitory oligonucleotides, the concentration of each of the inhibitory oligonucleotides present in the composition may be lower, e.g., in the range of 1-10 nM, such as 2-8 nM, 3-7 nM, 4-7 nm, 4-6 nM or 5 nM. Despite the lower concentration of the individual oligonucleotides, such a combination of at least two oligonucleotides may exhibit a combined knockdown efficiency that is comparable or even higher than when using only a single inhibitory oligonucleotide. It is noted that the skilled person is aware that the concentration of the inhibitory oligonucleotides present in a composition may also vary depending on the experimental setting of cell donor, transfection reagent, chemical modification, etc.

If the inhibitory oligonucleotide provided by the composition is an shRNA, the composition may optionally also comprise a delivery vehicle configured to express the shRNA in situ from a nucleic acid encoding said shRNA.

Such a delivery vehicle preferably is an expression vector encoding the inhibitory oligonucleotide as disclosed herein, i.e. it may be a minicircle, a plasmid, a cosmid, a phage, a virus or an artificial chromosome. Preferably, the expression vector is a viral vector, e.g., an adenoviral vector, a lentiviral vector or an adeno-associated viral (AAV) vector, most preferably an AAV vector.

The expression vector comprises an expression cassette, i.e. the necessary elements that permit transcription of a cargo nucleic acid sequence into the inhibitory oligonucleotide, e.g. the shRNA, such as a suitable promoter. In one embodiment, the expression vector may comprise a cargo sequence that encodes only a single inhibitory oligonucleotide, e.g. a single shRNA as disclosed herein. Alternatively, the expression vector may comprise a cargo sequence that encodes a plurality of different inhibitory oligonucleotides, e.g., any combination of the shRNAs disclosed herein. This way, it is possible to express multiple different shRNAs with different target sequences from a single expression vector.

In a preferred embodiment, the expression vector comprises a cargo sequence that encodes a combination of four different shRNAs capable of selectively binding, e.g. SEQ ID NO: 1, 3, 4 and 5 or SEQ ID NO: 2, 3, 4 and 5. Accordingly, the expression vector may encode, e.g., a first shRNA comprising SEQ ID NO: 22, a second shRNA comprising SEQ ID NO: 24, a third shRNA comprising SEQ ID NO: 25 and a fourth shRNA comprising SEQ ID NO: 26 or a first shRNA comprising SEQ ID NO: 23, a second shRNA comprising SEQ ID NO: 24, a third shRNA comprising SEQ ID NO: 25 and a fourth shRNA comprising SEQ ID NO: 26.

A person skilled in the art will be able to design suitable viral vectors to comprise a cargo sequence encoding one or more inhibitory oligonucleotides, e.g. one or more shRNAs based on general knowledge and the prior art. For example, a stretch of cargo DNA, i.e., a transgene, encoding the one or more inhibitory oligonucleotides, e.g., one or more shRNAs may be operably linked to a suitable promoter and inserted into the viral vector of choice. A transgene is considered to be "operably linked" to a promoter when said promoter is positioned in a correct functional location and/or orientation in relation to said transgene to control its transcription. The promoter used to express the one or more inhibitory oligonucleotides may be any constitutively active promoter known in the art, e.g., a Rous sarcoma virus (RSV) promoter, a human cytomegalovirus (CMV) promoter, an HIV promoter or a eukaryotic promoter such as e.g., EF1a, PGK1, UBC, or human beta actin. Alternatively, the promoter may be an inducible promoter that only drives expression of the one or more oligonucleotide molecules in the presence or absence of a certain stimulus. For example, the inducible promoter may be a Tet-regulated promoter, i.e., it may comprise a tetracycline response element (TRE) that can be bound by a tetracycline transactivator (tTA) protein in the presence of tetracycline or an analogue thereof, e.g., doxycycline. The promoter may also be a cell-specific promoter which only drives expression in a particular type of cell.

In another embodiment, the vector furthermore is a cell-specific vector capable of targeting the one or more oligonucleotide molecules, e.g. the one or more shRNAs or shRNAmiRs, to a cell or a tissue of interest, e.g., to cardiovascular cells or cells of the respiratory tract.

Accordingly, if the vector is a viral vector such as a lentiviral vector, the assembled viral vector is preferably pseudotyped, e.g., with one or more different viral envelope glycoproteins or a capsid to facilitate binding of the vector to the surface of the cell of interest. The term pseudotyping refers to the generation of viral vectors that carry foreign viral envelope glycoproteins on their surface or are encapsulated in a capsid of a different virus or viral serotype to specifically target only particular cell types of interest. The viral vector particles are usually assembled inside suitable packaging cells known in the art according to standard laboratory techniques before they are administered to the subject.

The present inventors have put particular focus on investigating the use of AVV vectors for delivering shRNA into human smooth muscle cells (SMC).

AAVs form the genus Dependoviruses within the family of parvoviruses (Parvoviridae), a family of single-stranded DNA viruses with a non-enveloped, icosahedral capsid. Their productive infection cycle with replication of AAV DNA and assembly of new infectious virus particles depends on the presence of a suitable helper virus, for example adenovirus or herpes simplex virus (HSV), hence its name and taxonomy classification. In the absence of helper viruses, AAV is able to establish a latent infection. *In vitro,* this often results in integration of the viral DNA into the genome of the culture cells, whereas, *in vivo,* the AAV DNA is thought to predominantly exist in an extrachromosomal state as a stable episome. So far, AAV could not be causally linked to a known disease in humans.

AAV vectors have shown excellent efficacy, a very good safety profile, high transduction rates and stable vector persistence combined with long-lasting expression in preclinical animal models and clinical trials for various monogenic diseases. These properties have boosted the use of AAV in gene transfer protocols for the treatment of a variety of genetic diseases, both in preclinical as well as in clinical studies.

The genome of the best characterized AVV serotype AAV-2 has a length of about 4.7 kb and contains as essential elements two open reading frames (ORFs) for the four regulatory (Rep) proteins Rep78, Rep68, Rep52 and Rep40 and the three capsid (Cap) proteins VP1, VP2 and VP3 flanked by two inverted terminal repeats (ITRs) of 145 bp each.

Currently, more than 12 different AAV serotypes and more than 100 naturally occurring capsid variants of AAV are known. A viral "serotype" is a variation within single virus species that are classified together based on their surface antigens, i.e., in the case of AAV, based on their serotype-specific viral capsids.

Serotypes AAV-2, -3, -5, -6 and -9 were first isolated from humans, serotypes AAV-4, -7, -8, -10, -11 and -12 were derived from monkeys and serotypes AAV-1 was isolated both from humans and monkeys. The distinct AAV serotypes may differ substantially in their tissue tropisms, i.e. they may target and infect distinct cell types and tissues. For instance, serotypes AAV-1, AAV-2, AAV-4, AAV-5 and AAV-8 are particularly suitable for transducing cells of the retina, whereas serotypes AAV-6 and -7 may be used for transducing skeletal muscle cells. The capsid of AAV serotype 9 enables AAV to bypass the blood-brain barrier (BBB) and is therefore a leading capsid for the transduction of the central nervous system (CNV) via systemic administration (Wang et al., 2019, Nat Rev Drug Discov.). The present inventors could confirm previous reports according to which the AAV serotype AAV-2 exhibits superior transduction efficiency in human smooth muscle cells compared to serotypes AAV-1, -3, -6, -8 or -9.

Thus, if the inhibitory oligonucleotide provided by the composition according to the invention is an shRNA that is to be delivered to smooth muscle cells, the composition preferably comprises an AAV vector of the AAV-2 serotype to express the shRNA in situ from a nucleic acid encoding said shRNA.

The expression vector used for delivering the oligonucleotide molecule, e.g. the shRNA, may also be a non-pathogenic, genetically modified bacterium or yeast comprising a prokaryotic or eukaryotic vector. The vector comprises a DNA molecule encoding the candidate oligonucleotide molecule operably linked to a promoter that controls expression of said candidate oligonucleotide molecule, as described above. The non-pathogenic bacterium is preferably invasive, i.e., it is capable of entering a host cell and may be derived, e.g., from a non-pathogenic strain of *Escherichia coli, Listeria, Yersinia, Rickettsia, Shigella, Salmonella, Legionella, Chlamydia, Brucella, Neisseria, Burkolderia, Bordetella, Borrelia, Coxiella, Mycobacterium, Helicobacter, Staphylococcus, Streptococcus, Vibrio, Lactobacillus, Porphyromonas, Treponema,* or *Bifidobacteriae.*

The prokaryotic or eukaryotic delivery vehicle may also be specifically designed for targeted delivery to a cell of interest by expressing a targeting moiety on its surface. The targeting moiety may be, e.g., a cell-specific antibody or ligand such as, e.g., a small peptide, polysaccharide or nucleic acid that can be bound by a receptor expressed on the surface of the cell of interest.

The different inhibitory oligonucleotides and compositions disclosed herein can be for scientific use, e.g., they may be used in *in vitro, ex vivo or in vivo* experiments that require effective knockdown of *ADAMTS7.* The compositions can, for example, be part of a kit that optionally includes additional components, such as cells, media, enzymes, cytokines, buffer solutions and/or a user manual.

However, in a preferred embodiment, the composition according to the invention may be for therapeutic use, i.e., the composition preferably is a pharmaceutical composition. Said pharmaceutical composition may comprise one or more additional excipients, e.g. a pharmaceutically acceptable carrier. Pharmaceutically acceptable carriers are well known in the art and may comprise aqueous solutions such as water or physiologically buffered saline or other solvents such as glycols, glycerol or oils. The term "pharmaceutically acceptable" refers those compounds, materials, compositions, and/or dosage forms that are, within the scope of sound medical judgement, suitable for use in contact with the tissues of the subject without causing excessive toxicity, irritation, allergic response or other problems or complications commensurate with a reasonable benefit/risk ratio.

In addition to the inhibitory oligonucleotides of the invention disclosed herein, the pharmaceutical composition may also provide or comprise other therapeutic inhibitory oligonucleotides already known in the prior art. If the pharmaceutical composition is to be used, for example, for the treatment of atherosclerosis or CAD, it may also provide or comprise, in addition to the one or more inhibitory oligonucleotides of the invention, a therapeutic ASO or siRNA, which targets, e.g., PCSK9. For example, in addition to the one or more inhibitory oligonucleotides of the invention, the pharmaceutical composition may also provide or comprise the FDA-approved inclisirane. The pharmaceutical composition may also, in addition to the inhibitory oligonucleotides of the invention described herein, provide or comprise a therapeutic inhibitory oligonucleotide which inhibits the production of apolipoprotein B-100. For example, this inhibitory oligonucleotide may be the FDA-approved ASO called Mipomersen (INN; trade name Kynamro).

The present invention further provides an implantable medical device, e.g., for keeping a blood vessel open, that comprises and has been configured to release one or more of the herein disclosed inhibitory oligonucleotides or compositions into its environment.

The medical device is implantable, i.e., it is intended for being inserted in a patient's body. It may be a stent or a balloon catheter, in particular, for use in treatment of a cardiovascular disease or disorder. Alternatively, it may also be an orthopaedic implant, e.g., for joint surgery or for treating bone fractures, osteoarthritis, scoliosis, spinal stenosis, and/or chronic pain.

A stent is a medical implant that is inserted into the lumen of an anatomic vessel or duct to keep the passageway open (https://en.wikipedia.org/wiki/Stent). It is usually a spiral wire prosthesis in the form of a tube made of metal or synthetic fibers with auxetic or mechanical properties for vasodilatation. As an alternative or in addition to the implantation of stents, inflatable balloon catheters have also been used for some time to expand the lumen of blood vessels.

The placement of stents or ballons is however associated with the risk of restenosis occurring. So-called "in-stent restenosis" develops in response to mechanical injury to the coronary artery during stent implantation and is accompanied by an inflammatory response. As a result, endothelial cells (ECs) begin to increase the expression of adhesion molecules on their surface, allowing leukocytes from the blood to adhere to them and enter the intima. This leads to an excessive proliferation of vascular smooth muscle cells (VSMCs) and a chronic inflammatory response that is maintained by the cascade of adhesion molecules (Hossfeld et al., 2013).

To prevent the development of restenosis, various types of drug-eluting stents (DESs) have been developed that release pharmacological agents capable of inhibiting the growth of neointimal tissue. Similarly, drug-coated balloons (DCBs) have been used to locally administer immunosuppressants such as rapamycin and cytostatics such as paclitaxel.

A sub-type of these DESs and DCBs include stents or balloons that are coated with inhibitory oligonucleotides such as siRNAs. This is achieved by incorporating, e.g., siRNAs that target genes known or suspected to drive restenosis into a polymeric matrix that is applied onto the surface of the stent or balloon. In doing so, it is possible to locally deliver these siRNAs to, e.g., endothelial cells of the vessel wall.

In some embodiments, the medical device, e.g., the stent or balloon catheter, is coated with the one or more inhibitory oligonucleotides, i.e., the one or more inhibitory oligonucleotides are immobilized on the outer surface of the medical device such as the stent or the balloon catheter.

Different methods of coating, i.e., immobilizing inhibitory oligonucleotides such as, e.g., siRNAs onto different surfaces, including the surfaces of stents, are well known in the art (Nolte et al., 2011, Hossfeld et al., 2013, Che et al., 2016, Koenig et al., 2017) and typically involve the application of coatings comprising or consisting of the inhibitory oligonucleotide complexed with a cationic polymer such as, e.g., polyethylenimine (PEI), hyaluronic-acid, chitosan or atelocollagen. As already explained herein, negatively charged inhibitory oligonucleotides can be complexed with such cationic polymers to form positively charged polyplexes that can be endocytosed by cells. To ensure the controlled and sustained release of these polyplexes and their stabilization on the stent, these polyplexes can be, e.g., embedded in suitable matrix formed of, e.g., gelatine on the stent surface. The inhibitory oligonucleotides may also be incorporated into thin films made of cationic polymers. These films may be, e.g., in the form of polyelectrolyte multilayers (PEMs) that are generated by using the well-established layerby-layer (LbL) technology first introduced by Decher et al., 1992. Further substrates suitable for coating inhibitory oligonucleotides to, e.g., stents, comprise, e.g., cationized pullulan hydrogels (Juan et al., 2009), dopamine (Joddar et al., 2013) or polyethylene glycol (PEG)-coated superparamagnetic iron oxide (SPIO) nanoparticles (Wang et al., 2024).

Methods for coating balloon catheters with siRNAs are also known from the prior art, e.g. from WO2012045469A1. Here, too, siRNA molecules can, e.g., be complexed with polyethyleneimine (PEI) as described herein, which are then embedded in a gelatine matrix on the balloon surface.

Alternatively, the medical device may also encompass the one or more inhibitory oligonucleotides of the invention, i.e. it may be configured to comprise or form a reservoir in which the inhibitory oligonucleotides of the invention are encapsulated and from which they are released into the environment after insertion of the medical device. Such medical devices are also known to those skilled in the art. For example, porous balloons have been used for some time to treat diseased vasculature, in which a therapeutic agent is infused into the inflatable interior of the porous balloon and through the porous wall of the balloon.

Again, in addition to the inhibitory oligonucleotides of the invention disclosed herein, the implantable medical device may also comprise and be configured to release other therapeutic inhibitory oligonucleotides already known in the prior art such as, e.g. a therapeutic ASO or siRNA which targets, e.g., PCSK9 like inclisirane and/or a therapeutic inhibitory oligonucleotide which inhibits the production of apolipoprotein B-100 such as Mipomersen (INN; trade name Kynamro).

In another embodiment, the medical device, e.g., the stent or a balloon catheter, releases a viral vector capable of expressing one or more of the herein disclosed shRNA in situ from a nucleic acid encoding said one or more shRNA. The viral vector preferably is an AAV vector of serotype AAV-2, as disclosed herein.

Again, the medical device, such as, e.g., the stent or ballon catheter, can either encompass the viral vector or, preferably, has an outer surface that is coated with the viral vector.

The coating of stents with a viral vector, in particular an AAV vector can be achieved as described in, e.g., Hooshdaran et al., 2022, which is herewith fully incorporated by reference. In brief, the protocol according to Hooshdaran et al., 2022 involves the use of cobalt/chromium stents and stainless steel mesh disks that were first cleaned with isopropanol and incubated in a 0.5% aqueous solution of polyallylamine bisphosphonate bearing latent thiol groups (PABT) at 72°C with shaking for 1 h followed by deprotection of thiol groups in the side chains of PABT with Tris(2-carboxyethyl)phosphine hydrochloride (TCEP) (12 mg/ml in 0.1 M acetate buffer at room temperature (RT) with shaking for 15 min). The samples are then extensively washed with degassed double-distilled water (DDW) and reacted under argon atmosphere with an 0.5% aqueous solution of polyethyleneimine with installed pyridyl-dithio groups (PEI(PDT)) at 28°C with shaking for 1 h, washed and incubated with thiolated protein G (PrG-SH; 125 µg/ml in degassed PBS) at 28°C with shaking for 1 h. The specimens are then washed with PBS and reacted with 50 µg/ml of an anti-AAV2 antibody with shaking at RT for 45 min. The samples are then washed in PBS and incubated with AAV2 suspension (1010 VG/ml in PBS) at RT with mild shaking for 45 min to immobilize the vector particles on the metal surface. Needless to say, the protocol described in Hooshdaran et al., 2022 is only one of many ways in which an AAV vector can be immobilised to a stent. The skilled person will be able to choose from other protocols reported in the literature, e.g., in Fishbein et al., 2017 or will be able to modify the protocol of Hooshdaran et al., 2022 by taking appropriate measures.

Viral vectors can be attached to a balloon catheter, e.g., by incorporating the viral vector in a suitable matrix formed, e.g., of a hydrogel polymer such as poly-carboxylic acids, cellulosic polymers, gelatine polyvinyl-pyrrolidone, maleic anhydride polymers, polyamides, polyvinyl alcohols, or polyethylene oxides and coating said matrix over the exterior surface of the balloon, as described, e.g., in Landau et al., 1995.

The herein disclosed inhibitory oligonucleotides, compositions and medical devices can be used in a method of treating a cardiovascular disease or condition such as atherosclerosis and atherosclerosis-induced neovascularization, restenosis, coronary artery disease (CAD) or thoracic aortic aneurysm and dissection (TAAD) in a subject in need thereof, wherein the method comprises administration of the inhibitory oligonucleotides, the pharmaceutical compositions or medical devices to the subject.

As described herein, the pathophysiological role of ADAMTS7 in atherosclerosis, restenosis and CAD is well-established and the present inventors could show that the herein disclosed inhibitory oligonucleotides can be used to successfully suppress ADAMTS7 expression in smooth muscle and endothelial cells, i.e., in cell types of particular significance during atherosclerosis. The inventors could also demonstrate in *in vitro assays,* that the ADAMTS7 knock-down using inhibitory oligonucleotides of the invention results in reduced SMC and EC migration and prevents angiogenesis, which serves as a marker of neovascularization during atherosclerosis.

The term "atherosclerosis-induced neovascularization" relates to the formation of new blood vessels, which can occur within atherosclerotic plaques. Neovascularisation in atherosclerosis can have a detrimental effect and lead to plaque instability and rupture. In addition, neovascularisation can promote inflammation and facilitate the recruitment of immune cells, further exacerbating plaque development and progression.

Moreover, work from others demonstrated elevated ADAMTS7 levels in plasma and the aorta during the early stages of thoracic aortic aneurysm and dissection (TAAD) in mice, highlighting the potential of ADAMTS7 as a biomarker for TAAD. Using an ADAMTS7 knockout mouse model, it could be shown that ADAMTS7 deficiency not only attenuates β-aminopropionitrile (BAPN)-induced murine TAAD but also improves overall survival (Gong et al., 2023). Thus, ADAMTS7 may also serve as a novel target for TAAD therapy that involves the use of the herein disclosed inhibitory oligonucleotides, pharmaceutical compositions and/or medical devices (Gong et al., 2023).

As described in more detail in the Examples below, the inventors found that lack of ADAMTS7 regulates the expression of various downstream markers involved, inter alia, in inflammation. The inventors thus further conducted an extensive literature search to identify intricate connections between elevated levels of ADAMTS7 and several additional diseases characterized by shared inflammatory processes.

A role of ADAMTS7 role was, e.g., described in rheumatoid arthritis (Liu et al., 2006). Recent in vivo evidence strongly indicates a pivotal role of ADAMTS7 also in the pathogenesis of inflammatory arthritis (Zhang et al., 2015). Moreover, in psoriatic patients, elevated serum levels of ADAMTS7 were also reported (Loyola et al., 2023; Karsulovic et al., 2023).

Accordingly, the inhibitory oligonucleotides, the compositions or medical devices of the present invention may also be for use in a method of treating an inflammatory disease selected from the group comprising rheumatoid arthritis, inflammatory arthritis and psoriasis in a subject in need thereof, wherein the method comprises administration of the inhibitory oligonucleotides, pharmaceutical compositions or medical devices to the subject.

The role of ADAMTS7 in lung adaptive immunity and inflammation has also yielded compelling evidence of its pivotal role in allergic airway disease (Jaiswal and Mishra, 2022). This positions ADAMTS7 as an attractive target for asthma treatment, offering novel avenues for managing this prevalent respiratory condition.

Moreover, recent genome-wide association studies have uncovered a robust association between ADAMTS7 and both chronic obstructive pulmonary disease (COPD) and lung cancer (unpublished data). These findings underscore the multifaceted impact of ADAMTS7 across various respiratory conditions, presenting new opportunities for therapeutic interventions.

Accordingly, the inhibitory oligonucleotides and compositions of the present invention may also be for use in a method of treating a pulmonary disease selected from the group consisting of asthma, chronic obstructive pulmonary disease (COPD) and lung cancer in a subject in need thereof, wherein the method comprises administration of the inhibitory oligonucleotides or the compositions to the subject.

In the context of the invention, "treating" a disease is to be understood to comprise a curative medical therapy of a subject with the intent to cure, ameliorate or stabilize a condition. The term "condition" refers to a disease, a syndrome, a disorder or a particular physiological state of an organism that is either manifested by distinct symptoms or can be diagnosed by using established markers capable of recognizing said state. In some embodiments, treating a condition is intended to mean that the progression of the condition is to be slowed, stopped or, preferably, reversed to allow for a perceivable improvement of the subject's well-being and health. Preventing a condition refers to precluding, averting, obviating, forestalling, stopping, or hindering said condition from happening in the first place. Preventing a condition thus also explicitly includes the prophylactic treatment of a subject.

The subject of the herein disclosed invention is preferably a human being. However, given that all of the target sequences of the herein disclosed inhibitory oligonucleotides are conserved in pigs, the subject may as well be a pig. In cases where an inhibitory oligonucleotide is used that selectively binds any of SEQ ID NO: 1, 2 or 3, the subject may also be a mouse, a rabbit or a rat. If the inhibitory oligonucleotide selectively binds SEQ ID NO: 3, the subject may also be a zebrafish.

Regardless of the specific medical indication, the herein disclosed inhibitory oligonucleotides or pharmaceutical compositions may be administered to a subject either systemically or locally. Where the inhibitory oligonucleotides or compositions according to the invention are to be used to treat, e.g., atherosclerosis, restenosis, coronary artery disease (CAD) or thoracic aortic aneurysm and dissection (TAAD), the inhibitory oligonucleotides and/or compositions of the invention may, e.g., be administered intravenously. Alternatively, the inhibitory oligonucleotides or compositions of the invention can be administered using the medical devices, e.g., the stents and /or balloon catheters as disclosed herein.

In the treatment of pulmonary diseases such as, asthma, chronic obstructive pulmonary disease (COPD) or lung cancer, local application of the inhibitory oligonucleotides or pharmaceutical compositions according to the invention is preferred. They may, e.g., be administered to the airways of a subject via inhalation, i.e., the pharmaceutical composition may be provided to the patient as an aerosol.

The herein disclosed inhibitory oligonucleotides or pharmaceutical compositions may, in some embodiments, be administered once. In alternative embodiments, multiple administrations of the inhibitory oligonucleotides or pharmaceutical compositions may be necessary to promote an improvement in the medical condition to be treated. For example, the inhibitory oligonucleotides or pharmaceutical compositions may need to be administered to the subject at least 2 times, at least 3 times, at least 4 times, at least 5 times, at least 10 times, at least 50 times, or at least 100 times before an improvement in the subject's condition occurs. In some embodiments, administration must occur regularly for as long as the subject's condition exists, possibly for a lifetime. The administration can be carried out by the subjects themselves or by medically trained specialists, e.g. a doctor.

The treatment of the subject with the inhibitory oligonucleotides or pharmaceutical compositions according to the invention can furthermore be combined with other therapeutic approaches. For instance, treatment of atherosclerosis, restenosis and/or CAD using the herein disclosed inhibitory oligonucleotides, compositions and medical devices may be combined with the administration of e.g., a HMG-CoA reductase inhibitor, e.g., one or more statins such as atorvastatin, fluvastatin, lovastatin, pitavastatin, pravastatin, rosuvastain or simvastatin, a PCSK9 inhibitor such as an antibody to PCSK9 or an inhibitory oligonucleotide targeting PCSK9 such as inclirisan and/or or a therapeutic inhibitory oligonucleotide which inhibits the production of apolipoprotein B-100 such as Mipomersen (INN; trade name Kynamro). Moreover, given the intricate association of ADAMTS7 with key pro-inflammatory pathways, notably IL-8-CXCR1/2, IL1-IL1ra, and TNFα-NFκB, therapies that involve ADAMTS7 knockdown using the herein described inhibitory oligonucleotides may be combined with the use of established inflammatory inhibitors targeting key components of these pathways, such as Reparixin, Lanraplenic, Anakinra, and Infliximab, to enhance treatment effectiveness and comprehensively address various facets of inflammatory processes. Similarly, treatment of lung cancer using the herein described inhibitory oligonucleotides and pharmaceutical compositions can be accompanied by conventional anti-cancer interventions such as chemotherapy or radiation therapy.

In particular, the invention provides the following embodiments:
1. An inhibitory oligonucleotide capable of selectively binding to a target nucleic acid sequence within an mRNA encoding human *ADAMTS7,* wherein the target nucleic acid sequence is selected from the group consisting of SEQ ID NO: 1, SEQ ID NO: 2, SEQ ID NO: 3, SEQ ID NO: 4, SEQ ID NO: 5, SEQ ID NO: 6 and SEQ ID NO: 7, and wherein the oligonucleotide molecule is selected from the group consisting of an RNA molecule capable of inducing RNAi and an antisense oligonucleotide (ASO).
2. The inhibitory oligonucleotide of embodiment 1, wherein the target nucleic acid sequence is SEQ ID NO: 1.
3. The inhibitory oligonucleotide of embodiment 1, wherein the target nucleic acid sequence is SEQ ID NO: 2.
4. The inhibitory oligonucleotide of embodiment 1, wherein the target nucleic acid sequence is SEQ ID NO: 3.
5. The inhibitory oligonucleotide of embodiment 1, wherein the target nucleic acid sequence is SEQ ID NO: 4.
6. The inhibitory oligonucleotide of embodiment 1, wherein the target nucleic acid sequence is SEQ ID NO: 5.
7. The inhibitory oligonucleotide of embodiment 1, wherein the target nucleic acid sequence is SEQ ID NO: 6.
8. The inhibitory oligonucleotide of embodiment 1, wherein the target nucleic acid sequence is SEQ ID NO: 7.
9. The oligonucleotide molecule of any of embodiments 1-8, wherein the inhibitory oligonucleotide is an RNA molecule capable of inducing RNAi selected from the group comprising an siRNA or an shRNA.
10. The inhibitory oligonucleotide of embodiment 9, wherein the inhibitory oligonucleotide is an siRNA.
11. The inhibitory oligonucleotide of embodiment 10, wherein the antisense strand of the siRNA comprises a nucleic acid sequence having at least 90% sequence identity to any of SEQ ID NO: 8, 10, 12, 14, 16, 18 or 20.
12. The inhibitory oligonucleotide of embodiment 10 or 11, wherein the antisense strand of the siRNA comprises a nucleic acid sequence having at least 90% sequence identity to any of SEQ ID NO: 8.
13. The inhibitory oligonucleotide of embodiment 10 or 11, wherein the antisense strand of the siRNA comprises a nucleic acid sequence having at least 90% sequence identity to any of SEQ ID NO: 10.
14. The inhibitory oligonucleotide of embodiment 10 or 11, wherein the antisense strand of the siRNA comprises a nucleic acid sequence having at least 90% sequence identity to any of SEQ ID NO: 12.
15. The inhibitory oligonucleotide of embodiment 10 or 11, wherein the antisense strand of the siRNA comprises a nucleic acid sequence having at least 90% sequence identity to any of SEQ ID NO: 14.
16. The inhibitory oligonucleotide of embodiment 10 or 11, wherein the antisense strand of the siRNA comprises a nucleic acid sequence having at least 90% sequence identity to any of SEQ ID NO: 16.
17. The inhibitory oligonucleotide of embodiment 10 or 11, wherein the antisense strand of the siRNA comprises a nucleic acid sequence having at least 90% sequence identity to any of SEQ ID NO: 18.
18. The inhibitory oligonucleotide of embodiment 10 or 11, wherein the antisense strand of the siRNA comprises a nucleic acid sequence having at least 90% sequence identity to any of SEQ ID NO: 20.
19. The inhibitory oligonucleotide of any of embodiments 10-18, wherein the antisense strand of the siRNA consists of a nucleic acid sequence having at least 90% sequence identity to any of SEQ ID NO: 8, 10, 12, 14, 16, 18 or 20.
20. The inhibitory oligonucleotide of any of embodiments 10-19, wherein the antisense strand of the siRNA comprises a nucleic acid sequence having at least 95% sequence identity to any of SEQ ID NO: 8, 10, 12, 14, 16, 18 or 20.
21. The inhibitory oligonucleotide of any of embodiments 10-20, wherein the antisense strand of the siRNA consists of a nucleic acid sequence having at least 95% sequence identity to any of SEQ ID NO: 8, 10, 12, 14, 16, 18 or 20.
22. The inhibitory oligonucleotide of any of embodiments 10-21, wherein the antisense strand of the siRNA comprises a nucleic acid sequence having 100% sequence identity to any of SEQ ID NO: 8, 10, 12, 14, 16, 18 or 20.
23. The inhibitory oligonucleotide of any of embodiments 10-22, wherein the antisense strand of the siRNA consists of a nucleic acid sequence having 100% sequence identity to any of SEQ ID NO: 8, 10, 12, 14, 16, 18 or 20.
24. The inhibitory oligonucleotide of embodiment 9, wherein the inhibitory oligonucleotide is an shRNA.
25. The inhibitory oligonucleotide of embodiment 24, wherein the shRNA comprises a nucleic acid sequence having at least 90% sequence identity to any of SEQ ID NO: 8, 10, 12, 14, 16, 18 or 20.
26. The inhibitory oligonucleotide of any of embodiments 24 or 25, wherein the shRNA comprises a nucleic acid sequence having at least 90% sequence identity to SEQ ID NO: 8.
27. The inhibitory oligonucleotide of any of embodiments 24 or 25, wherein the shRNA comprises a nucleic acid sequence having at least 90% sequence identity to SEQ ID NO: 10.
28. The inhibitory oligonucleotide of any of embodiments 24 or 25, wherein the shRNA comprises a nucleic acid sequence having at least 90% sequence identity to SEQ ID NO: 12.
29. The inhibitory oligonucleotide of any of embodiments 24 or 25, wherein the shRNA comprises a nucleic acid sequence having at least 90% sequence identity to SEQ ID NO: 14.
30. The inhibitory oligonucleotide of any of embodiments 24 or 25, wherein the shRNA comprises a nucleic acid sequence having at least 90% sequence identity to SEQ ID NO: 16.
31. The inhibitory oligonucleotide of any of embodiments 24 or 25, wherein the shRNA comprises a nucleic acid sequence having at least 90% sequence identity to SEQ ID NO: 18.
32. The inhibitory oligonucleotide of any of embodiments 24 or 25, wherein the shRNA comprises a nucleic acid sequence having at least 90% sequence identity to SEQ ID NO: 20.
33. The inhibitory oligonucleotide of any of embodiments 24-32 wherein the shRNA comprises a nucleic acid sequence having at least 95% sequence identity to any of SEQ ID NO: 8, 10, 12, 14, 16, 18 or 20.
34. The inhibitory oligonucleotide of any of embodiments 24-33, wherein the shRNA comprises a nucleic acid sequence having 100% sequence identity to any of SEQ ID NO: 8, 10, 12, 14, 16, 18 or 20.
35. The inhibitory oligonucleotide of embodiment 24-34, wherein the shRNA comprises a nucleic acid sequence having at least 90% sequence identity to any of SEQ ID NO: 22-28.
36. The inhibitory oligonucleotide of any of embodiments 24 or 35, wherein the shRNA comprises a nucleic acid sequence having at least 90% sequence identity to SEQ ID NO: 22.
37. The inhibitory oligonucleotide of any of embodiments 24 or 35, wherein the shRNA comprises a nucleic acid sequence having at least 90% sequence identity to SEQ ID NO: 23.
38. The inhibitory oligonucleotide of any of embodiments 24 or 35, wherein the shRNA comprises a nucleic acid sequence having at least 90% sequence identity to SEQ ID NO: 24.
39. The inhibitory oligonucleotide of any of embodiments 24 or 35, wherein the shRNA comprises a nucleic acid sequence having at least 90% sequence identity to SEQ ID NO: 25.
40. The inhibitory oligonucleotide of any of embodiments 24 or 35, wherein the shRNA comprises a nucleic acid sequence having at least 90% sequence identity to SEQ ID NO: 26.
41. The inhibitory oligonucleotide of any of embodiments 24 or 35, wherein the shRNA comprises a nucleic acid sequence having at least 90% sequence identity to SEQ ID NO: 27.
42. The inhibitory oligonucleotide of any of embodiments 24 or 35, wherein the shRNA comprises a nucleic acid sequence having at least 90% sequence identity to SEQ ID NO: 28.
43. The inhibitory oligonucleotide of any of embodiments 24-42 wherein the shRNA comprises a nucleic acid sequence having at least 95% sequence identity to any of SEQ ID NO: 22-28.
44. The inhibitory oligonucleotide of any of embodiments 24-43, wherein the shRNA comprises a nucleic acid sequence having 100% sequence identity to any of SEQ ID NO: 22-28.
45. The inhibitory oligonucleotide of embodiment 1-8, wherein the inhibitory oligonucleotide is an antisense oligonucleotide (ASO).
46. The inhibitory oligonucleotide of embodiment 45, wherein the ASO comprises a nucleic acid sequence that corresponds to a subsequence of at least 6 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20.
47. The inhibitory oligonucleotide of any of embodiments 45 or 46, wherein the ASO comprises a nucleic acid sequence that corresponds to a subsequence of at least 6 nucleotides of SEQ ID NO: 8.
48. The inhibitory oligonucleotide of any of embodiments 45 or 46, wherein the ASO comprises a nucleic acid sequence that corresponds to a subsequence of at least 6 nucleotides of SEQ ID NO: 10.
49. The inhibitory oligonucleotide of any of embodiments 45 or 46, wherein the ASO comprises a nucleic acid sequence that corresponds to a subsequence of at least 6 nucleotides of SEQ ID NO: 12.
50. The inhibitory oligonucleotide of any of embodiments 45 or 46, wherein the ASO comprises a nucleic acid sequence that corresponds to a subsequence of at least 6 nucleotides of SEQ ID NO: 14.
51. The inhibitory oligonucleotide of any of embodiments 45 or 46, wherein the ASO comprises a nucleic acid sequence that corresponds to a subsequence of at least 6 nucleotides of SEQ ID NO: 16.
52. The inhibitory oligonucleotide of any of embodiments 45 or 46, wherein the ASO comprises a nucleic acid sequence that corresponds to a subsequence of at least 6 nucleotides of SEQ ID NO: 18.
53. The inhibitory oligonucleotide of any of embodiments 45 or 46, wherein the ASO comprises a nucleic acid sequence that corresponds to a subsequence of at least 6 nucleotides of SEQ ID NO: 20.
54. The inhibitory oligonucleotide of any of embodiments 45-53, wherein the ASO comprises a nucleic acid sequence that corresponds to a subsequence of at least 10 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20.
55. The inhibitory oligonucleotide of any of embodiments 45-54, wherein the ASO comprises a nucleic acid sequence that corresponds of a subsequence of at least 12 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20.
56. The inhibitory oligonucleotide of any of embodiments 45-55, wherein the ASO comprises a nucleic acid sequence that corresponds to a subsequence of at least 14 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20.
57. The inhibitory oligonucleotide of any of embodiments 45-56, wherein the ASO comprises a nucleic acid sequence that corresponds to a subsequence of at least 16 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20.
58. The inhibitory oligonucleotide of any of embodiments 45-46, wherein the ASO consists of a nucleic acid sequence of any of SEQ ID NO: 29, SEQ ID NO: 30, SEQ ID NO: 31, SEQ ID NO: 32, SEQ ID NO: 33, SEQ ID NO: 34 or SEQ ID NO: 35.
59. The inhibitory oligonucleotide of any of embodiments 45-46 and 58, wherein the ASO consists of a nucleic acid sequence of SEQ ID NO: 29.
60. The inhibitory oligonucleotide of any of embodiments 45-46 and 58, wherein the ASO consists of a nucleic acid sequence of SEQ ID NO: 30.
61. The inhibitory oligonucleotide of any of embodiments 45-46 and 58, wherein the ASO consists of a nucleic acid sequence of SEQ ID NO: 31.
62. The inhibitory oligonucleotide of any of embodiments 45-46 and 58, wherein the ASO consists of a nucleic acid sequence of SEQ ID NO: 32.
63. The inhibitory oligonucleotide of any of embodiments 45-46 and 58, wherein the ASO consists of a nucleic acid sequence of SEQ ID NO: 33.
64. The inhibitory oligonucleotide of any of embodiments 45-46 and 58, wherein the ASO consists of a nucleic acid sequence of SEQ ID NO: 34.
65. The inhibitory oligonucleotide of any of embodiments 45-46 and 58, wherein the ASO consists of a nucleic acid sequence of SEQ ID NO: 35.
66. The inhibitory oligonucleotide of any of embodiments 1-8 and 45-65, wherein the ASO is selected from the group comprising a DNA antisense oligonucleotide and a gapmer.
67. The inhibitory oligonucleotide of embodiment 66, wherein the ASO is a DNA antisense oligonucleotide.
68. The inhibitory oligonucleotide of embodiment 66, wherein the ASO is a gapmer.
69. A composition providing a first inhibitory oligonucleotide of any of embodiments 1-68.
70. The composition of embodiment 69, further providing a second inhibitory oligonucleotide selected from an inhibitory oligonucleotide of any of embodiments 1-68, wherein the second inhibitory oligonucleotide is different from the first inhibitory oligonucleotide.
71. The composition of embodiment 70, further providing a third inhibitory oligonucleotide selected from an inhibitory oligonucleotide of any of embodiments 1-68, wherein the third inhibitory oligonucleotide is different from the first and the second inhibitory oligonucleotides.
72. The composition of embodiment 71, further providing a fourth inhibitory oligonucleotide selected from an inhibitory oligonucleotide of any of embodiments 1-68, wherein the fourth inhibitory oligonucleotide is different from the first, the second and the third inhibitory oligonucleotides.
73. The composition of embodiment 72, further providing a fifth inhibitory oligonucleotide selected from an inhibitory oligonucleotide of any of embodiments 1-68, wherein the fifth inhibitory oligonucleotide is different from the first, the second, the third and the fourth inhibitory oligonucleotides.
74. The composition of embodiment 73, further providing a sixth inhibitory oligonucleotide selected from an inhibitory oligonucleotide of any of embodiments 1-68, wherein the sixth inhibitory oligonucleotide is different from the first, the second, the third, the fourth and the fifth inhibitory oligonucleotides.
75. The composition of embodiment 74, further providing a seventh inhibitory oligonucleotide selected from an inhibitory oligonucleotide of any of embodiments 1-68, wherein the seventh inhibitory oligonucleotide is different from the first, the second, the third, the fourth, the fifth and the sixth inhibitory oligonucleotides.
76. The composition of any of embodiments 70-75, wherein the first, the second and optionally, the third, the fourth, the fifth, the sixth and the seventh inhibitory oligonucleotides are different in that they selectively bind to different target sequences of any of SEQ ID NO: 1, 2, 3, 4, 5, 6 or 7 and/or are different types of inhibitory oligonucleotides selected from the group consisting of siRNAs, shRNAs and ASOs.
77. The composition of any of embodiments 69-76, wherein the composition comprises the first and, optionally, the second, the third, the fourth, the sixth and the seventh oligonucleotides.
78. The composition of any of embodiments 69-77, further comprising a delivery vehicle that comprises the first and, optionally, the second, the third, the fourth, the sixth and the seventh oligonucleotides or is associated with the first and, optionally, the second, the third, the fourth, the sixth and the seventh oligonucleotides.
79. The composition of embodiment 78, wherein the delivery vehicle is:
   1. a liposomal or an exosomal carrier,
   2. a lipid nanoparticle,
   3. a bacterial minicell,
   4. an extracellular vesicle,
   5. a cationic polymer selected from the group comprising polyethylenimine (PEI) or DEAE-dextran, or
   6. lipophilic moiety selected from the group comprising a palmityl- or cholesterol-residue.
80. The composition of any of embodiments 69-76, wherein the inhibitory oligonucleotide is an shRNA of any of embodiments 1-8 or 24-44, wherein the composition comprises a delivery vehicle configured to express the shRNA from a nucleic acid encoding said shRNA.
81. The composition of embodiment 80, wherein the delivery vehicle configured to express the shRNA from a nucleic acid encoding said shRNA is
   a) a viral vector, or
   b) non-pathogenic, genetically modified bacterium or yeast comprising a prokaryotic or eukaryotic expression vector,
82. The composition of any of embodiments 80 or 81, wherein the delivery vehicle configured to express the shRNA from a nucleic acid encoding said shRNA is an AAV vector of the AAV-2 serotype.
83. The composition of any of embodiments 69-82, wherein the composition is a pharmaceutical composition, optionally comprising a pharmaceutically acceptable carrier.
84. The composition of embodiment 83, further comprising an inhibitory oligonucleotide capable of inhibiting PCSK9, e.g., Inclirisan.
85. The composition of any of embodiments 83 or 84, further comprising an inhibitory oligonucleotide capable of inhibiting apolipoprotein B-100, e.g., Mipomersen.
86. An implantable medical device, wherein the implantable medical device comprises and is capable of releasing one or more oligonucleotides according to any of embodiments 1 to 68 or a composition according to any of embodiments 69 to 85.
87. The implantable medical device of embodiment 86, wherein the implantable medical device further comprises and is capable of releasing an inhibitory oligonucleotide capable of inhibiting PCSK9, e.g., Inclirisan.
88. The implantable medical device of any of embodiments 86 or 87, wherein the implantable medical device further comprises and is capable of releasing an inhibitory oligonucleotide capable of inhibiting apolipoprotein B-100, e.g., Mipomersen.
89. The implantable medical device of any of embodiments 86-88, wherein the implantable medical device is able to keep a blood vessel open.
90. The implantable medical device of any of embodiments 86 or 89, wherein the implantable medical device is a stent or a balloon catheter.
91. The implantable medical device of any of embodiments 86-88, wherein the implantable medical device is an orthopaedic implant.
92. The implantable medical device of any of embodiments 86-91, wherein the implantable medical device is coated with the one or more oligonucleotides according to any of embodiments 1 to 68 or a composition according to any of embodiments 69 to 85.
93. The implantable medical device of any of embodiments 86- 91, wherein the implantable medical device is coated with a viral vector configured to express one or more shRNAs of any of embodiments 1-8 or 24-44 from a nucleic acid encoding said one or more shRNAs.
94. The implantable medical device of embodiment 86, wherein the implantable medical device is coated with a composition according to embodiment 82.
95. The inhibitory oligonucleotide of any of embodiments 1 to 68, the composition of any of embodiments 69 to 85 or the medical device of any of embodiments 86-90 and 92-94 for use in a method of treating a cardiovascular disease or disorder selected from the group consisting of atherosclerosis and atherosclerosis-induced neovascularization, coronary artery disease (CAD) and thoracic aortic aneurysm and dissection (TAAD), wherein, optionally, the treatment is combined with a HMG-CoA reductase inhibitor selected from the group of statins comprising atorvastatin, fluvastatin, lovastatin, pitavastatin, pravastatin, rosuvastain and simvastatin, and/or a PCSK9 inhibitor selected from the group comprising an antibody to PCSK9 and inclirisan and/or the inhibitor of apolipoprotein B-100 Mipomersen .
96. The inhibitory oligonucleotide of any of embodiments 1 to 68, the composition of any of embodiments 69-85 or the implantable medical device of any of embodiments 86 and 81 and 91-94 for use in a method of treating an inflammatory disease selected from the group comprising inflammatory arthritis and psoriasis.
97. The inhibitory oligonucleotide of any of embodiments 1 to 68 or the composition of any of embodiments 69-85 for use in a method of treating pulmonary disease selected from the group consisting of asthma, chronic obstructive pulmonary disease (COPD) and lung cancer.

Throughout the invention, the term "about" is intended to be understood as "+/- 10%". If "about" relates to a range, it refers to both lower and upper limits of the range. "A" is intended to mean "one or more", if not explicitly mentioned otherwise.

All literature cited herein is herewith fully incorporated. The present invention is further illustrated, but not limited, by the following examples.

**Table 1: List of Sequences**

| SEQ ID NO: | Sequence name |
|---|---|
| 1 | target sequence of phu-siADAMTS7-6 |
| | CAGTTTTGGCATTCAGCATGACG |
| 2 | target sequence of phu-siADAMTS7-5 |
| | CACAGTTTTGGCATTCAGCATGA |
| 3 | target sequence of phu-siADAMTS7-7 |
| | CATGTCTCCACAGCTCCTGTACG |
| 4 | target sequence of phu-siADAMTS7-1 |
| | TCCAACGAGGACTACTTCATTGA |
| 5 | target sequence of phu-siADAMTS7-2 |
| | GCAGAAAAGCATCAACATGAAGG |
| 6 | target sequence of phu-siADAMTS7-3 |
| | AAGATGAGGAGGAGGACCTAAAG |
| 7 | target sequence of phu-siADAMTS7-4 |
| | TGAGGAGGAGGACCTAAAGATCA |
| 8 | antisense strand of phu-siADAMTS7-6 |
| | TCATGCTGAATGCCAAAACtg |
| 9 | sense strand of phu-siADAMTS7-6 |
| | GTTTTGGCATTCAGCATGAcg |
| 10 | antisense strand of phu-siADAMTS7-5 |
| | ATGCTGAATGCCAAAACTGtg |
| 11 | sense strand of phu-siADAMTS7-5 |
| | CAGTTTTGGCATTCAGCATga |
| 12 | antisense strand of phu-siADAMTS7-7 |
| | TACAGGAGCTGTGGAGACAtg |
| 13 | sense strand of phu-siADAMTS7-7 |
| | TGTCTCCACAGCTCCTGTAcg |
| 14 | antisense strand of phu-siADAMTS7-1 |
| | AATGAAGTAGTCCTCGTTGga |
| 15 | sense strand of phu-siADAMTS7-1 |
| | CAACGAGGACTACTTCATTga |
| 16 | antisense strand of phu-siADAMTS7-2 |
| | TTCATGTTGATGCTTTTCTgc |
| 17 | sense strand of phu-siADAMTS7-2 |
| | AGAAAAGCATCAACATGAAgg |
| 18 | antisense strand of phu-siADAMTS7-3 |
| | TTAGGTCCTCCTCCTCATCtt |
| 19 | sense strand of phu-siADAMTS7-3 |
| | GATGAGGAGGAGGACCTAAag |
| 20 | antisense strand of phu-siADAMTS7-4 |
| | ATCTTTAGGTCCTCCTCCTca |
| 21 | sense strand of phu-siADAMTS7-4 |
| | AGGAGGAGGACCTAAAGATca |
| 22 | guide sequence of phu-shADAMTS7-6 (shRNA) |
| | CGTCATGCTGAATGCCAAAACTG |
| 23 | guide sequence of phu-shADAMTS7-5 (shRNA) |
| | TCATGCTGAATGCCAAAACTGT |
| 24 | guide sequence of phu-shADAMTS7-7 (shRNA) |
| | GTACAGGAGCTGTGGAGACATG |
| 25 | guide sequence of phu-shADAMTS7-1 (shRNA) |
| | CAATGAAGTAGTCCTCGTTGGA |
| 26 | guide sequence of phu-shADAMTS7-2 (shRNA) |
| | CTTCATGTTGATGCTTTTCTGC |
| 27 | guide sequence of phu-shADAMTS7-3 (shRNA) |
| | TTTAGGTCCTCCTCCTCATCTT |
| 28 | guide sequence of phu-shADAMTS7-4 (shRNA) |
| | GATCTTTAGGTCCTCCTCCTCA |
| 29 | phu-asADAMTS7-6 (ASO) |
| | ATGCTGAATGCCAAAACTG |
| 30 | phu-asADAMTS7-5 (ASO) |
| | ATGCTGAATGCCAAAACTGTG |
| 31 | phu-asADAMTS7-7 (ASO) |
| | TACAGGAGCTGTGGAGACATG |
| 32 | phu-asADAMTS7-1 (ASO) |
| | AATGAAGTAGTCCTCGTTGGA |
| 33 | phu-asADAMTS7-2 (ASO) |
| | TTCATGTTGATGCTTTTCTGC |
| 34 | phu-asADAMTS7-3 (ASO) |
| | ATGGTGCGTGATCTTTAGGT |
| 35 | phu-asADAMTS7-4 (ASO) |
| | ATGGTGCGTGATCTTTAGGT |
| 36 | Commercially available siRNA against hsADAMTS7 |
| | GCACCUGUGGAGUGCAAG |
| 37 | siRNA targeting mouse Adamts7 1 |
| | GCGGGTAGCCAGACGGTAA |
| 38 | siRNA targeting mouse Adamts7 2 |
| | GCAGCGATCAGTCAGCAAA |
| 39 | phu-siADAMTS7_miss |
| | AGAAGAGCACCAATATGCAGG |

### Brief description of the Drawings

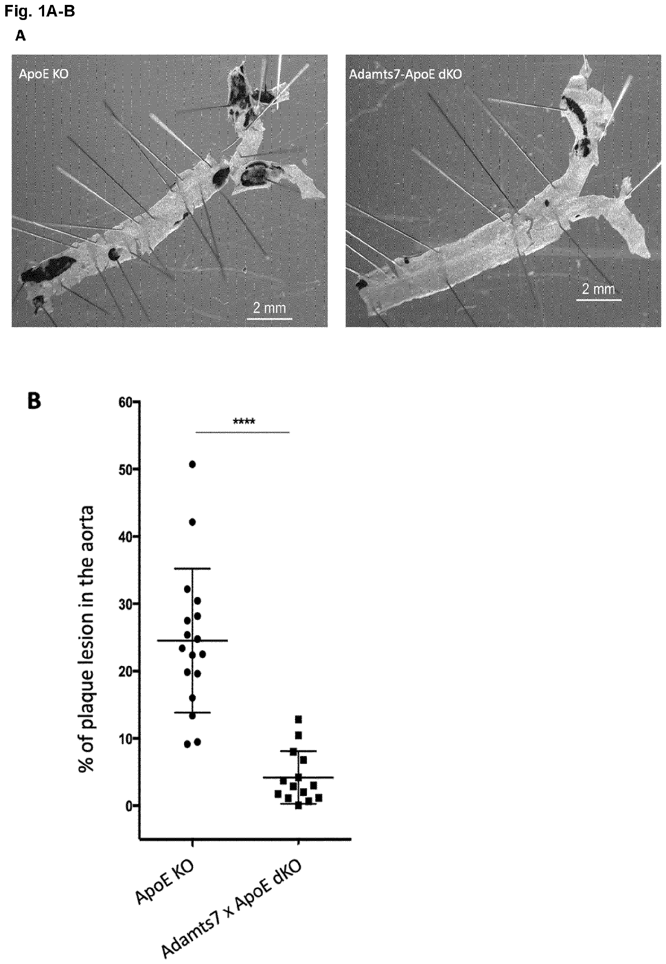

**Figure 1****:** Lack of ADAMTS7 reduces atherosclerosis in spontaneous ApoE -/- atherogenic mouse model. Representative Oil Red O-stained aortas (black) from 60 weeks old males (A). The percentage of plaque deposits in the aorta is quantified (B). P < 0.05 was considered statistically significant. For all tests: *, P < 0.05; **, P < 0.01; ***, P < 0.001.

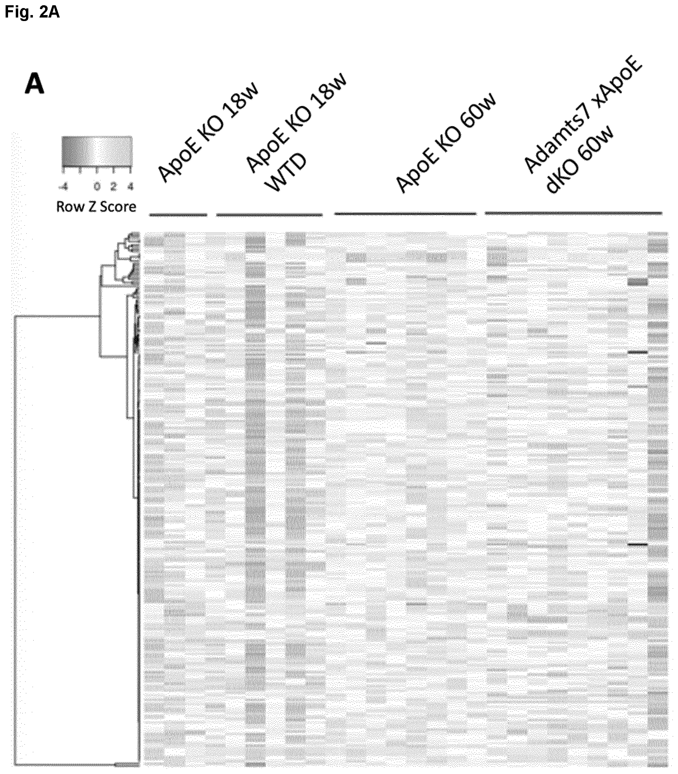

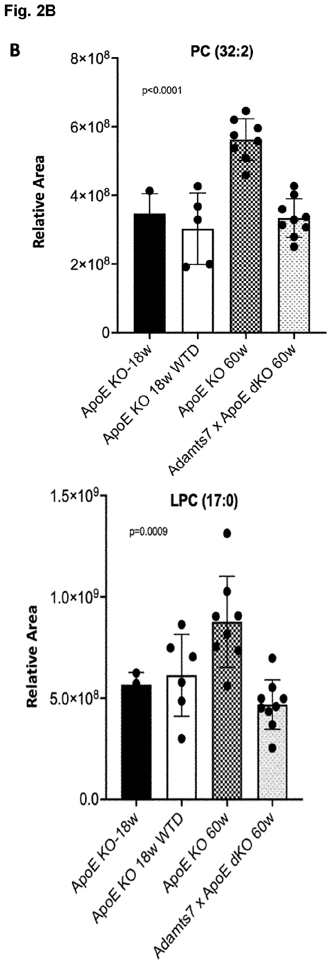

**Figure 2****:** Lipid Profiling. A) Heatmap illustrating variations in lipid species levels among different groups: young atherogenic ApoE KO mice at 18 weeks on a western-type diet (ApoE (-/-) 18w), corresponding controls (ApoE (-/-) 18w), aged mice at 60 weeks (ApoE (-/-) 60 w), and mice lacking ADAMTS7 (Adamts7 (-/-) x ApoE (-/-) 60 w). B) Highlighted are two distinct lipid species, PC (32:2) and LPC (17:0), exhibiting significant variations between the groups.

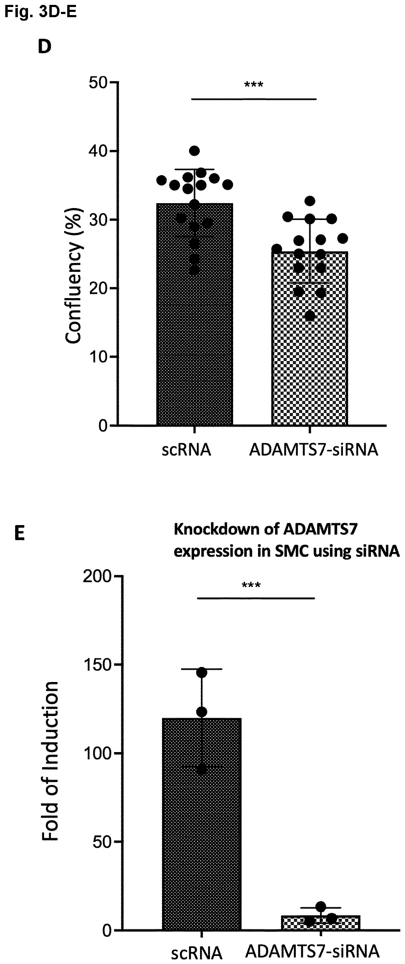

**Figure 3****:** ADAMTS7 Downregulation studies in human SMCs. A) Single cell RNAseq analysis of ADAMTS7 gene expression in different atherosclerosis cell types (www.plaqview.com). B) TNFa stimulates the gene expression of ADAMTS7 and vice versa. This in turn stimulates the NF-kB signaling pathway. ADAMTS7 siRNA downregulation in SMC demonstrated a decrease in the expression of inflammation, Adhesion, and ECM markers. C-D) ADAMTS7 KD leads to a decrease in SMC migration E) Successful Knockdown of ADAMTS7 in SMCs using siRNA.. P < 0.05 was considered statistically significant. For all tests: *, P < 0.05; **, P < 0.01; ***, P < 0.001. *: created by smart.ser-vier.com

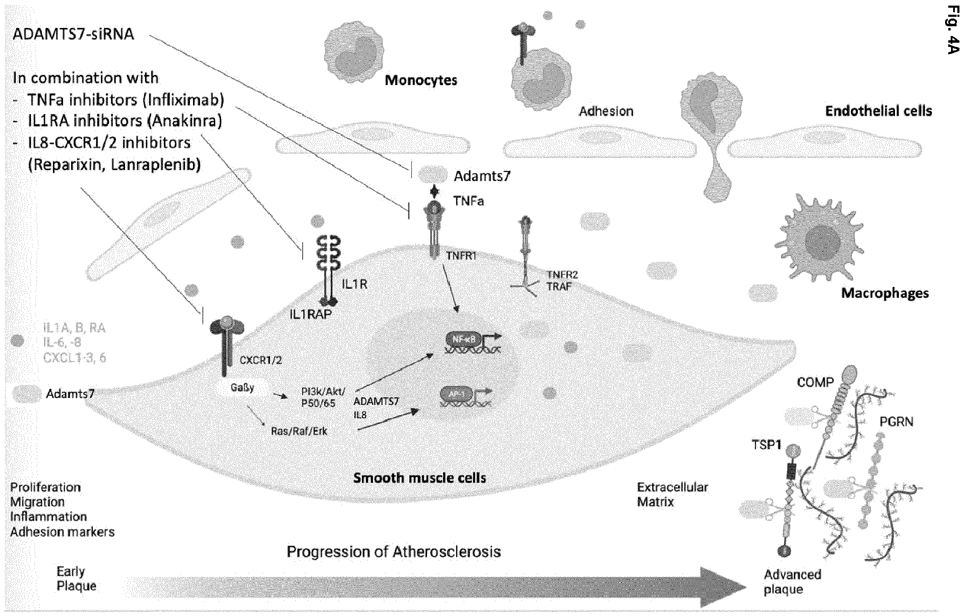

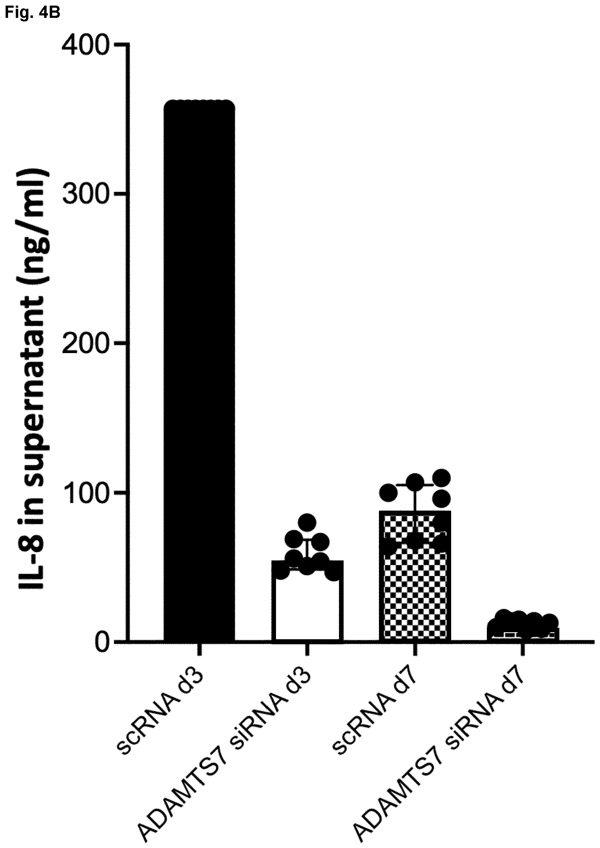

**Figure 4****:** Comprehensive therapeutic strategy targeting ADAMTS7-mediated pathways. A) The figure depicts a comprehensive therapeutic strategy that capitalizes on the downstream regulation of ADAMTS7 to address critical pathways implicated in atherosclerosis and restenosis. Initiated by TNFα through NFκB, this cascade regulates inflammation, proteases, and extracellular matrix degradation - all pivotal to atherosclerosis. This encompasses processes like SMC migration and proliferation. IL-8-CXCR1/2 and TNFα-NFκB pathways further amplify or interact with this sequence. Additionally, studies suggest a positive feedback loop between ADAMTS-7 and TNFα as presented in A. Our results demonstrated a reduction in IL-8 secretion upon ADAMTS7 knockdown at day 3 (d3) and day 7 (d7) post transfection using the siRNA target sequence described in section 7.3 (B). Created with Biorender.com

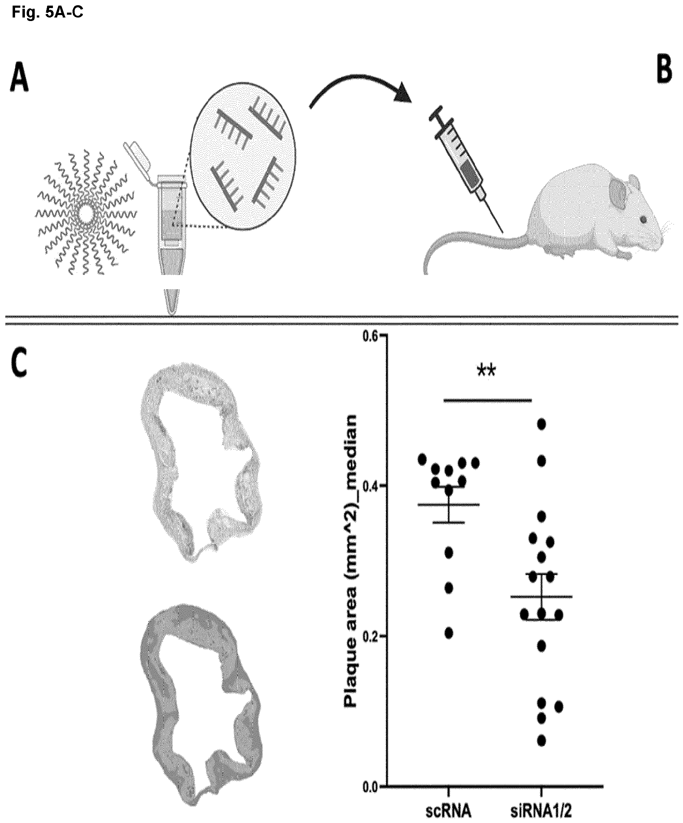

**Figure 5****:** Successful nanoparticle-siRNA approach to treat atherosclerosis in mice. Male mice were injected with siRNA targeting Adamts7 gene (siRNA1/2), three times between 30 and 45 weeks of age (A-B). As control we used scramble siRNA (scRNA). Mice were sacrificed at the age of 45 weeks and the percentage of plaque deposits in red in the aortic root is quantified (C). P < 0.05 was considered statistically significant. For all tests: *, P < 0.05; **, P < 0.01; ***, P < 0.001. Created with Biorender.com

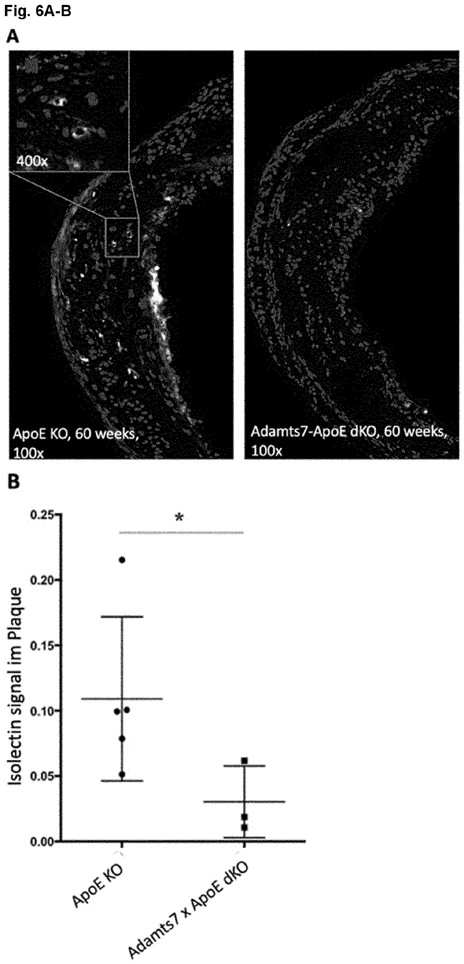

**Figure 6****:** Neovascularization within atherosclerotic plaques. Neovascularization was visualized through green fluorescence labeling with Isolectin-FITC (shown in white). Cell nuclei (in gray) were highlighted using DAPI. A) Sections of the aortic root with atherosclerotic plaques from mice lacking Adamts7 on an atherogenic ApoE background (Adamts7 (-/-) x ApoE (-/-)) and controls, ApoE KO, were stained with Isolectin and DAPI. B) Isolectin staining was quantified and normalized to DAPI. P < 0.05 was considered statistically significant. For all tests: *, P < 0.05; **, P < 0.01; ***, P < 0.001.

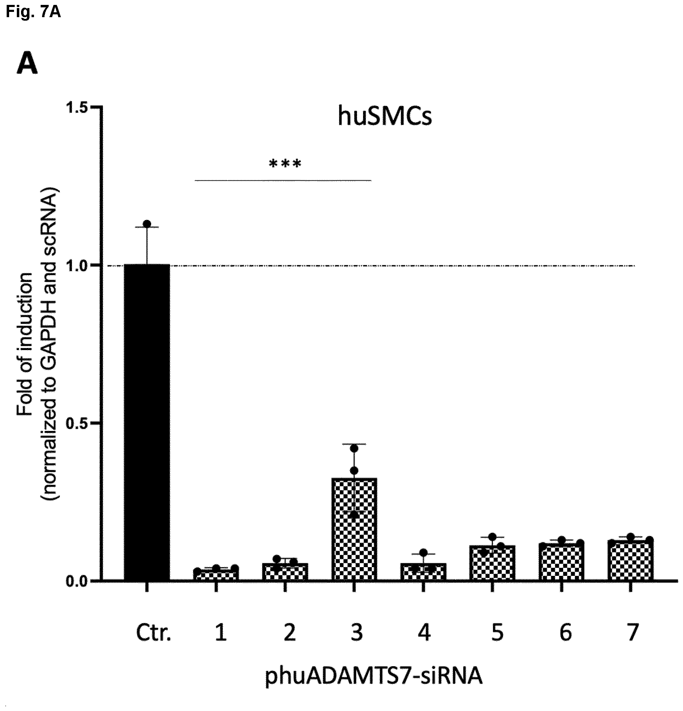

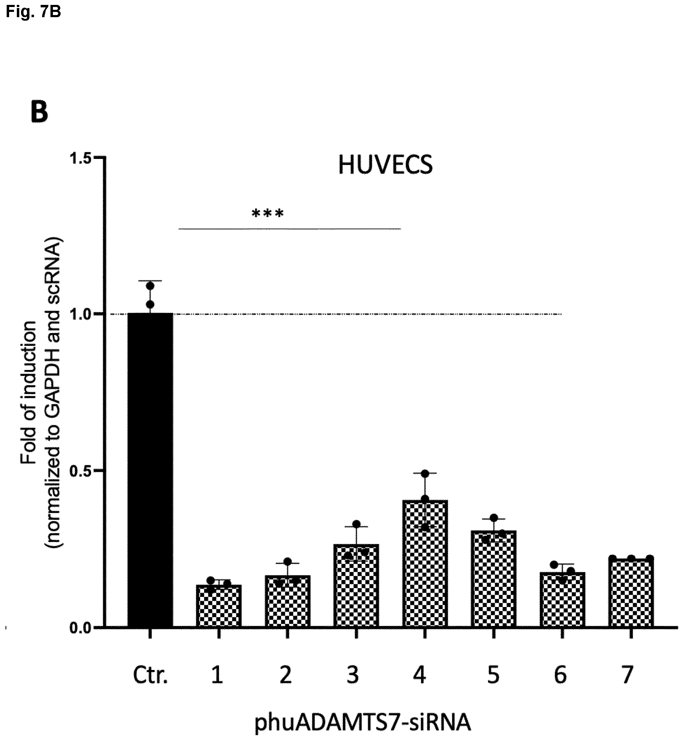

**Figure 7****:** In-vitro validation of the seven selected phu-ADAMTS7 siRNAs. In order to validate the efficiency of the selected phu-ADAMTS7 siRNAs (1-7), an in-vitro assessment was conducted using qPCR analysis in human smooth muscle cells (huSMCs) and human umbilical vein endothelial cells (HUVECs). The knockdown efficacy of ADAMTS7 was evaluated by comparing it to control siRNAs (Ctr. and Scr.) in huSMCs (A) and HUVECs (B). The delta-delta-Ct method was employed for the relative quantification of gene expression, using GAPDH as an internal standard gene. *, P < 0.05; **, P < 0.01; ***, P < 0.001.

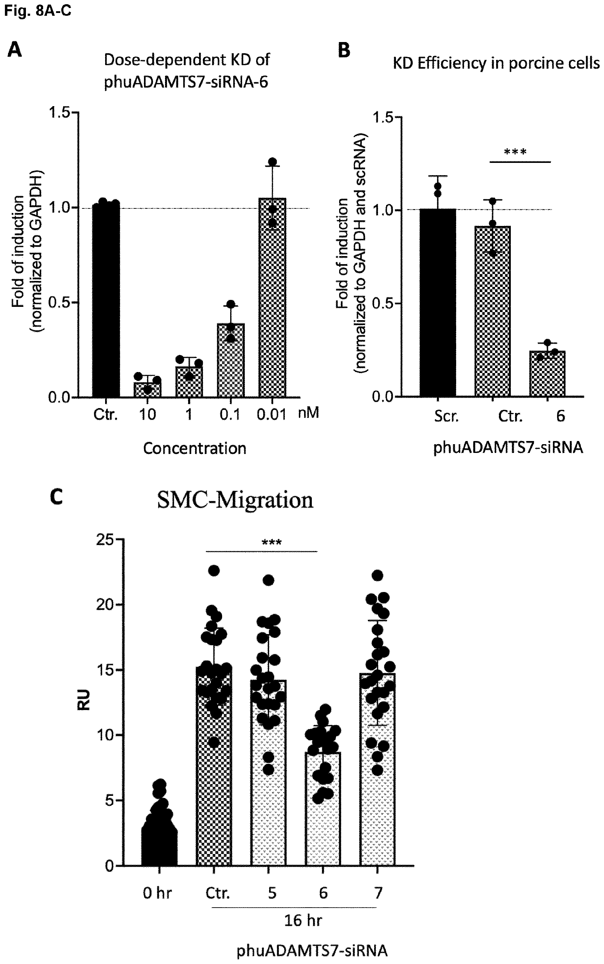

**Figure 8****:** Knockdown of ADAMTS7 in human and porcine cells and its impact on SMC migration. A) The knockdown efficiency of phuADAMTS7-6 siRNAs was initially tested in human aortic SMCs using different concentrations. B) Significant knockdown of Adamts7 was observed in porcine cells using phuADAMTS7-6. C) An SMC migration assay was employed to assess the functional biological effect of the three siRNAs. Values are presented in relative units (RU) and normalized to the initial number of cells at time point zero. A P value of < 0.05 was considered statistically significant for all tests: *, P < 0.05; **, P < 0.01; ***, P < 0.001.

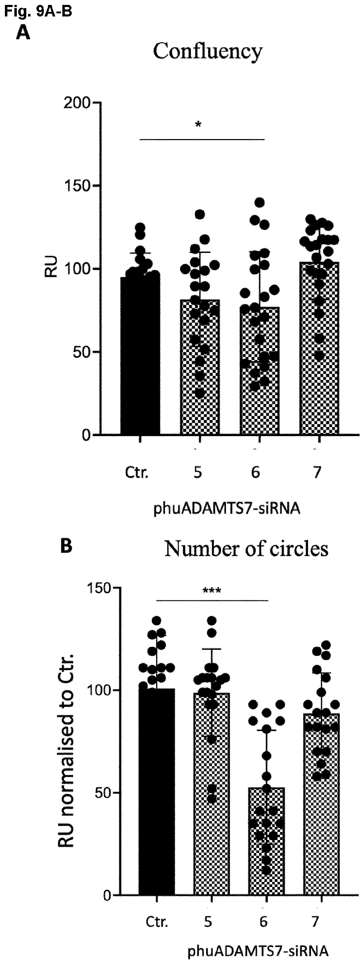

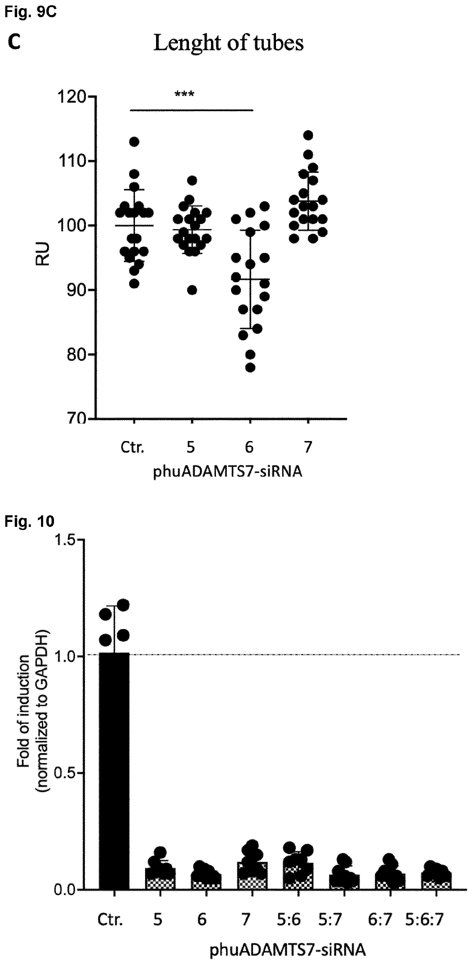

**Figure 9****:** Functional impact of ADAMTS7 knockdown (KD) on tube formation parameters in HUVECs. The functional impact was evaluated through a tube formation assay in HUVEC cells. Various parameters were analyzed, including cell confluency (A), the number of formed circles (B), and tube length (C). Values are expressed in relative units (RU) and normalized to the initial cell count at time point zero. A P value of < 0.05 was considered statistically significant for all tests: *, P < 0.05; **, P < 0.01; ***, P < 0.001.
**Figure 10****:** Combinations of low-concentration siRNA. Various combinations of the three lead siRNAs were employed to evaluate their collective effect on ADAMTS7 gene expression knockdown in human SMCs, employing qPCR analysis. The knockdown efficacy of ADAMTS7 was assessed against control siRNAs (Ctr.), and the relative quantification of gene expression was conducted using delta-delta-Ct method, with GAPDH serving as the internal standard gene. Single siRNAs were used at a concentration of 10 nM each. For dual siRNA combinations, 5 nM each was employed. Similarly, for the combination of all three siRNAs, a concentration of 3.33 nM each was utilized.

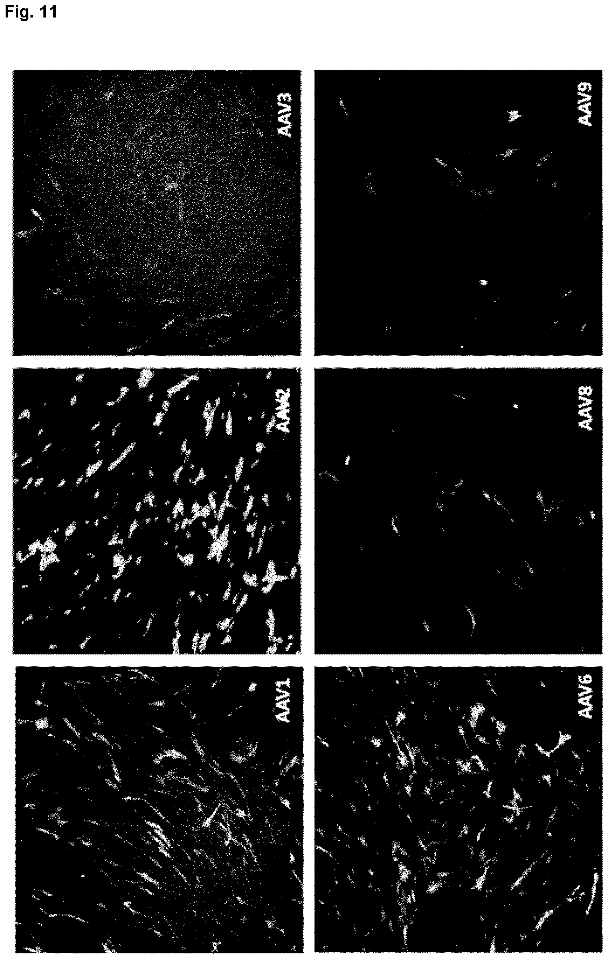

**Figure 11****:** Transduction in Human Smooth Muscle Cells using various AAV serotypes. Different AAV serotypes (AAV-1, 2, 3, 6, 8, 9) carrying the enhanced green fluorescent protein (EGFP) were used to assess the transduction efficiency in human SMCs. The expression of EGFP (white) serves as a visual representation of the experimental setup and outcomes.

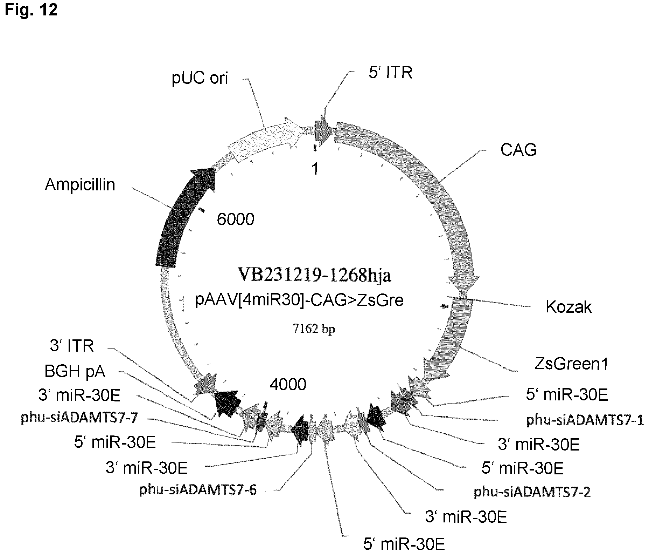

**Figure 12****:** Use of a Multi-shRNA Vector. A multi-shRNA vector, the pAAV-4miR30 Vector, is strategically designed to augment the silencing efficiency. The vector serves as a sophisticated tool for providing a targeted and robust approach to precisely modulate the expression of the ADAMTS7 gene. The incorporation of ZsGreen1 serves as a visual marker to facilitate the visualization of transduction efficiency.

### Examples:

### Example 1:

In the following example, the inventors have examined the significance of ADAMTS7 in atherosclerosis as well as atherosclerosis-associated conditions and the emergence of CAD.

### 1. Lack of ADAMTS7 protects aged mice from atherosclerosis

Atherosclerosis is a disease of aging; indeed, increasing age is an independent risk factor for development of the condition. A recent study using ultrasound imaging demonstrated a rapid increase of plaque deposits in individuals between the age of 40- and 50-years (Lopez-Melgar et al., 2020). We made similar observations by histological analysis and quantification in the proatherogenic ApoE knockout (ApoE KO) mouse models, which rapidly develop atherosclerotic deposits between the age of 25 and 40 weeks (data not shown). We generated Adamts7 knockout mice on this proatherogenic ApoE KO genetic background and studied spontaneous atherosclerosis progression at middle-age (60 weeks) without feeding a western type diet. Our data demonstrated a dramatic prevention of atherosclerosis in Adamts7 KO x ApoE KO double knockout (Adamts7-ApoE dKO) mice relative to ApoE KO control mice (Figure 1 A-B).

### 2. ADAMT7 deficiency mitigates atherosclerosis-associated high-risk lipid metabolites and improves health

Atherosclerosis is widely believed to be caused by disorders of lipid metabolism. We first aimed to investigate the impact of atherosclerosis susceptibility on plasma lipid metabolites by comparing atherogenic ApoE KO mice to non-atherogenic wild type C57BL/6 mice. Mass spectrometry analysis was performed to profile 234 lipid species in plasma samples from both groups. As expected, we observed significant changes in lipid profiles between the atherogenic and non-atherogenic mice, consistent with previously published data.

Thereafter we evaluated the dietary impact and compared lipid profiles in plasma samples between young mice (18 weeks) fed a western-type diet (WTD) and those on a normal chow diet. Notable differences were not observed between the two groups.

To further examine the effect of age, we compared plasma samples from 18-week-old and mid-aged 60-week-old ApoE-/- mice. The results, illustrated in Figure 2A, revealed substantial changes in lipid profiles with age, particularly in mid-aged (60 weeks) compared to young (18 weeks) mice. Notably, approximately 50% of the observed changes were associated with the phosphatidylcholine pathway.

Building on this data, we investigated whether the observed athero-protective effect in ApoE KO mice lacking Adamts7 described above could be attributed to changes in lipid metabolites. Interestingly, we discovered dramatic alterations in lipid profiles with age. Remarkably, the absence of ADAMTS7 in aged mice reversed these changes to levels comparable to those observed in young mice. The effect for specific phosphatidylcholine (PC) (32:2) and lyso-phosphatidylcholine (LPC) (17:0) blood profiles is illustrated in Figure 2B.

Our findings suggest that these specific phosphatidylcholine species could potentially serve as biomarkers for human aging, and their inhibition through ADAMTS7 knockdown may have the potential to improve human health. This finding aligns with recent research that identified an association between an ADAMTS7 genetic variant (rs3825807) and longevity, potentially through the promotion of healthy lipid metabolism (Zhang et al., 2020). Thus, future studies are needed to find the association between rs3825807 and our identified mouse lipid profiles in human.

### 3. Silencing of ADAMTS7 impacts inflammation, ECM, and adhesion markers, and SMC migration

ADAMTS7 is strongly associated with coronary artery atherosclerotic disease (Schunkert et al., 2011; Reilly et al., 2011). Single cell RNAseq analysis of coronary arteries demonstrated that ADAMTS7 is expressed predominantly in SMCs, using the interactive web application PlaqView (Ma et al, 2022) (www.plaqview.com) (Figure 3A), consistent with previous work showing that ADAMTS7 is expressed and localized to vascular smooth muscle in human atherosclerotic plaques (Pu et al., 2013). Thus, SMCs are the atherosclerosis-related cell type of choice for studying the regulation and function of ADAMTS7 in the context of CAD, particularly its role in expression of inflammatory, ECM markers as well as ECM degradation markers. We established an in vitro assay to study the role of ADAMTS7 in human SMCs using an siRNA targeting the following sequence GCACCUGUGGAGUGCAAG (Ambion, SEQ ID NO: 36) to knockdown (KD) ADAMTS7 gene expression (Figure 3E). Furthermore, we tested the effects of different atherosclerosis-related stimuli on ADAMTS7 gene regulation, and demonstrated stimulation of ADAMTS7 gene expression by TNF-alpha (TNFa) (Figure 3B). Using different gene expression arrays, we also showed that lack of ADAMTS7 regulates the expression of various downstream markers involved in inflammation, adhesion and ECM (Figure 3B). In more detail we found that lack of ADAMTS7 decreases the expression of Inflammatory markers (ILLA, IL1B, IL1RA; and CXCL1, CXCL2, CXCL3, CXCL6, CXCL8). We validated the downregulation of CXCL8 (IL8) using western blot and ELISA analysis in supernatant media (Figure 4B). Additionally, an enhancement in extracellular matrix (ECM) production was observed, involving COL11A1, COL14A1, and COL15A1, achieved through the upregulation of ITGA7 and ITGA8 signal transmission. As consequence these changes led to reduced migration of SMCs lacking ADAMTS7 relative to scRNA controls (Figure 3C-D). Overall, our data suggest that ADAMTS7 participates in the development and progression of atherosclerosis via increasing inflammation and SMC migration. This observation suggests a dual benefit of the intervention, not only by mitigating inflammation but also by promoting tissue repair and integrity.

The data presented above provide compelling evidence that the downstream regulation of ADAMTS7 plays a pivotal role in several critical pathways, including inflammation, extracellular matrix remodeling, and the IL-8-CXCR1/2 and TNFα-NFκB pathways. The interaction between ADAMTS7 and TNFα triggers a sequence of events that significantly contribute to atherosclerosis progression, specifically through mechanisms involving smooth muscle cell migration or proliferation and phenotype switching. Through the targeted modulation of IL-8-CXCR1/2 and TNFα-NFκB pathways, there exists a promising avenue to suppress inflammation and mitigate extracellular matrix degradation - both of which are central to the development of atherosclerosis and restenosis. By synergizing the impact of ADAMTS7-KD with interventions aimed at these pathways, including the use of TNFa and CXCR1/2 inhibitors, as well as repurposing inflammatory mechanisms, a potential therapeutic strategy emerges. Additionally, augmenting this approach with lipid-lowering agents such as statins and PCSK9 inhibitors enriches its scope, thereby effectively addressing atherosclerosis and restenosis pathogenesis. Figure 4 illustrates and summarizes this strategic approach.

### 4. Successful therapy using a nanoparticle-siRNA approach to inhibit ADAMTS7 and prevent atherosclerosis in mice

All animal experiments were approved by the German animal studies committee of Schleswig-Holstein. Based on our previous studies showing rapid spontaneous atherosclerotic deposits in mice between the age of 25 and 40 weeks, we initiated a pilot study to test whether we could inhibit progression of atherosclerosis during this period of time by targeting Adamts7 gene expression using each of the two siRNA targeting mouse Adamts7 sequence (siRNA 1: GCGGGTAGCCAGACGGTAA (SEQ ID NO: 37 and siRNA2: GCAGCGATCAGTCAGCAAA SEQ ID NO: 38), commercially available siRNA (Thermofischer)) at 30 weeks-of-age. A non-viral nanoparticle approach was used to deliver the siRNA into target cells. Mice aged 30 weeks were injected with the nanoparticle-siRNA complex three times with 4-weeks interval between each of the three injections (Figure 5A-B). At 45 weeks old, mice were sacrificed and the extent of atherosclerosis at the aortic root was evaluated after histological staining with Oil-red O in red; 8 to 10 sections around the three heart valves were selected per animal and plaque areas were calculated (Figure 5C). As shown in Figure 5C, mice treated with both siRNAs (siRNA1/2) displayed less atherosclerosis than those treated with scrambled control RNA (scRNA).

### 5. Adamts7 deficiency leads to decrease plaque neovascularization

Neovascularization, the formation of new blood vessels, can occur within atherosclerotic plaques. Neovascularization in atherosclerosis can have detrimental effects leading to plaque instability and rupture. Further, neovascularisation may promote inflammation and facilitate the recruitment of immune cells, further exacerbating plaque development and progression. Inhibiting excessive angiogenesis, may hold potential for future therapeutic interventions in atherosclerosis. We examined the link between lack of ADAMTS7 and neovascularization in atherogenic ApoE KO mice. ApoE KO mice lacking Adamts7 (Adamts7-ApoE dKO) and ApoE mice as controls were utilized at 60 weeks of age, as detailed above. Hearts, including aortas, were dissected, rid of fat and connective tissues, and subsequently fixed in 4% PFA. Cryosectioning produced 8-10 µm sections of the aortic root. To evaluate neovascularization in atherosclerotic plaques, we employed fluorescent markers for endothelial cells (Isolectin IB4), depicted in white in Figure 6A. DAPI (4',6'-diamino-2-phenylindole) was used for blue nuclei staining. Subsequently, images were captured, and Isolectin signals in plaques were quantified as displayed in Figure 6B. The results presented in Figure 6B indicate reduced neovascularization within the plaques of Adamts7 x ApoE dKO mice compared to ApoE controls, suggesting a protective effect against plaque progression and destabilization.

### Example 2

Based on the published data and the above-described experiments, it is evident that inhibiting ADAMTS7 has the potential to be safe and beneficial in treating CAD. The inventors thus proposed to reach ADAMTS7 inhibition through siRNA. As will be shown in the following Example, the present inventors employed bioinformatic analysis, to specifically design and select effective lead siRNAs within conserved regions across species. These lead siRNAs, which demonstrate sequence similarities between human and commonly used laboratory animal models, have been thoroughly tested and validated using atherosclerotic-related cells, such as SMCs and endothelial cells, to evaluate the biological impact of *ADAMTS7* gene and its product.

### 1. Use of computational analysis to select lead siRNA that can be used in both human and animal studies

siRNAs bind specifically to complementary mRNA, targeting specific gene sequences and silencing their expression. Initially, we designed 1,027 siRNAs covering the entire human ADAMTS7 cDNA sequence (NM_014272) using four publicly available online tools: RNAxs, siDirect v2.0, i-Score Designer, and OligoWalk. The 1,027 siRNAs underwent further computational analysis to identify the most effective ones based on prediction scores. Selection criteria including target similarity (off-target), GC content, secondary structure, RNA-RNA interaction, efficacy, as well as molecular modeling and docking analysis, were applied to predict lead siRNAs.

In order to find siRNAs with similar sequence homology between humans and pigs, which are recognized as good preclinical animal models, we obtained pig transcript sequences for ADAMTS7 (XM_021098838.1 and XM_021098839.1) and aligned them with the human transcript sequence using MAFFT. We identified 31 conserved regions that were suitable for siRNA design with similar sequences between human and pig. Seven siRNAs were found to have similar sequences between human and pig (phu-siADAMTS7-1, -2, -3, -4, -5, -6 and - 7, corresponding to SEQ ID NO: 14, 16, 18, 20, 10, 8 and 12, respectively). To extend using these sequences for other commonly used laboratory animal models, we aligned these identified sequences with the mouse sequence and discovered that only three siRNAs (phu-si-ADAMTS7 -5, -6 and -7 (corresponding to SEQ ID NO: 10, 8 and 12, respectively) exhibited similarity among human (hu), pig (p), and mouse (m) within exon 8 in a conserved protein domain of Zinc-dependent metalloprotease. From the three lead siRNAs two (phu-si-ADAMTS7 -5 and -6) targeted the same sequence with a 2-base pair displacement within exon 8.

Thus, we identified unique ADAMTS7 siRNA that is conserved across rodent and non-rodent species, and thus more likely to be conserved across human population.

To ensure appropriate experimental controls, we also designed an siRNA with 4 mismatches to a phu-siADAMTS7-miss (AGAAGAGCACCAATATGCAGG, SEQ ID NO: 39), which was used as an internal negative control (Ctr.) in our study. Additionally, a commercially available scramble siRNA (Scr.) (ThermoFischer scientific Cat# 4390843) was employed as a second negative control.

### 2. In-vitro-functional assessment of the Knockdown Efficacy of the Lead In-silico Selected siRNAs

### 2.1 Validation of ADAMTS7 knockdown in atherosclerosis relevant cells SMCs and endothelial cells

To affirm the effectiveness of siRNAs in downregulating ADAMTS7 gene expression, atherosclerosis-relevant cell types - including smooth muscle cells (SMCs) and human umbilical vein endothelial cells (HUVECs) - were employed. Both SMCs and HUVECs are relevant for functional assays including migration and angiogenesis, respectively. After transfection with the selected in silico-designed and selected seven siRNAs, the ADAMTS7 gene expression was evaluated through qPCR, using the delta-delta Ct method and normalizing to GAPDH as the internal reference gene. Relative quantification was performed using control siRNAs (Ctr. or Scr.) (Figure 7).

We initially evaluated the performance of the seven selected siRNAs in SMCs. The results demonstrate that all tested siRNAs significantly reduced ADAMTS7 gene expression in SMCs compared to the mismatch control after 48 hours post-transfection (Figure 7A). Notably, phu-siADAMTS7-1, -2, and -4 achieved robust knockdown, with residual expression levels below 6% (phu-siADAMTS7-1: 4% ± 0.8 %; phu-siADAMTS7-2: 6% ± 1.2 %; phu-siADAMTS7-4: 6% ± 2.9 %). Similarly, phu-siADAMTS7-5, -6, and -7 also demonstrated strong knockdown, with residual expression levels ranging from 11% to 14% (phu-siADAMTS7-5: 11% ± 2.4 %; phu-siADAMTS7-6: 12% ± 0.8 %; phu-siADAMTS7-7: 14% ± 1.1%). However, phu-siADAMTS7-3 exhibited lower knockdown efficiency (33% ±10.7%).

Notably, our approach identified lead siRNAs with superior knockdown efficiency compared to those previously described by Dr. Patrick Michael Schindler in his PhD thesis (https://me-diatum.ub.tum.de/doc/1543434/1543434.pdf). Dr. Schindler's work employed commercially available siRNAs in SMCs, resulting in the following knockdown percentages after 48 hours of transfection: s22050: 13.8 ± 5.0 %; s22051: 29.2 ± 6.6 %; s22052: 29.0 ± 10.5 %. Importantly, these knockdown percentages did not lead to significant differences in SMC migration. Therefore, it became evident that achieving a knockdown efficiency with residual expression levels below 14% is crucial to observe any functional impact.

In summary, all of our in-silico designed lead siRNAs, except for phu-siADAMTS7-3, demonstrated robust knockdown with residual expression levels below 14% in SMCs compared to the mismatch control. Building on these findings, we further assessed the performance of the seven siRNAs in HUVEC cells, cell type important for angiogenesis assay, which yielded similar results. Notably, phu-siADAMTS7-1, -2, -6, and -7 exhibited particularly strong knockdown performance in HUVECs (Figure 7B).

Our focus then shifted to siRNAs with shared target sequences across mouse, pig, and human, with special attention to three specific siRNAs located within the conserved protein domain of the Zinc-dependent metalloprotease in exon 8. The knockdown efficacy of these three siRNAs was initially evaluated in human cells (Figure 7A). Remarkably, all three siRNAs exhibited substantial knockdown efficiency compared to the mismatch control, with remarkable performance below 14%. Especially we further examined phu-siADAMTS7-6 and established a correlation between varying siRNA concentrations and knockdown efficiency in human SMCs (Figure 8A).

To further validate the knockdown efficacy across species mouse, pig, and human, phu-siADAMTS7-6 was tested in porcine cells (Figure 8B). As anticipated, a notable reduction in ADAMTS7 gene expression was observed in porcine cells, aligning with the expected knockdown pattern.

### 2.2 Silencing of ADAMTS7 and its impacts on SMC migration

To functionally investigate its impact on atherosclerosis-related parameters, we employed the smooth muscle cell (SMC) migration assay. The objective was to assess the influence of ADAMTS7 gene expression knockdown (KD) on SMC migration using wound-healing assays conducted in a 12-well plate format with ibidi 4-well culture inserts. Approximately 2.2 × 10⁴ SMCs per well were seeded into insert wells and incubated at 37°C and 5% CO₂ for 24 hours. Following overnight siRNA transfection with each of the three lead siRNAs (phu-siADAMTS7-5, -6, -7), cells were incubated in M231 culture medium supplemented with Smooth Muscle Differentiation Supplement (SMDS), containing 1% (v/v) fetal bovine serum (FBS) and 30 µg/mL heparin. After 48 hours, inserts were removed, and cells were washed with phosphate-buffered saline and placed in M231 SMDS medium supplemented with 5 ng/mL PDGF-BB to stimulate cell migration. Subsequent images captured at 0 and 12 hours using an Olympus IX70 microscope were analyzed using an in-house Python script. The mean pixel distances were calculated relative to time 0. The results, as depicted in Figure 8C, revealed a notable reduction in cell migration for phu-siADAMTS7-6 and -7 siRNAs compared to the mismatch control (Ctr.). Particularly, the knockdown of ADAMTS7 utilizing phu-siADAMTS7-6 demonstrated a robust inhibition of SMC migration compared to the control siRNA.

### 2.3 Silencing of ADAMTS7 impacts EC migration and inhibits angiogenesis a marker of neovascularization in atherosclerosis

Neovascularization within atherosclerotic plaques can destabilize them, promoting inflammation and immune cell recruitment, making it a potential target for therapeutic interventions in atherosclerosis. Endothelial cells (ECs) play a pivotal role in angiogenesis. In vitro angiogenesis assays offer advantages of speed, cost-effectiveness, and interpretability. These assays are based on the concept that ECs cultured on a supportive matrix form tubule-like structures. Among these assays, the Matrigel-based assay, utilizing a matrix from murine tumors, has become the most prevalent method for studying in vitro tubule formation (Donovan, et al., 2001). To study tube formation in vitro we used Human vascular endothelial cells (HUVECs; single donor; Promocell C-12200, Lot. 0113005). We first seeded HUVECs at approximately 7 000 cells per cm² in ECGM2 (Endothelial Cell Growth Medium 2, Ready-to-use; Promocell C-22011). The cells were passaged at -90% confluency. To investigate the impact of ADAMTS7 knockdown on tube formation, we seeded cells in 6-Well plates at a density of 25,000 cells per cm² 24 hours prior to siRNA transfection. The transfection complex consisted of Opti-MEM and Oligofectamine-siRNA, conducted in serum-free ECGM2 (Promocell C-22210) for 3 hours. siRNA transfection involved 30 nM concentration of each of the specific siRNAs, along with Ctr. as a negative control. Subsequently, the medium was changed to ECGM2 with supplements. The following day, cells were labeled with 2 µM CellTracker in Opti-MEM for 30 minutes. Ultimately, cells were observed under a microscope and various angiogenesis parameters were quantified including cell confluency, Tubes length, tubes number (circle numbers). As shown in the Figure 9 phuADAMTS7-6 performed best. This goes in line with its strong impact on SMC migration. The observed reduction in angiogenesis or neovascularization resulting from the lack of ADAMTS7 holds important implications for atherosclerosis. Angiogenesis, the formation of new blood vessels, is closely linked to the progression of atherosclerosis, a chronic inflammatory condition affecting arteries. The finding that knocking down ADAMTS7, especially using phuADAMTS7-6, resulted in favorable angiogenesis outcomes highlights the potential role of ADAMTS7 in regulating angiogenic processes. This suggests that ADAMTS7 might promote angiogenesis, which in turn is associated with the destabilization of atherosclerotic plaques and the potential for rupture, leading to adverse cardiovascular events. However, it is vital to consider that the effects of inhibiting angiogenesis could vary in different physiological contexts. While reducing angiogenesis might benefit atherosclerosis by reducing plaque size and enhancing plaque stability, it could also influence processes like wound healing and tissue repair, where angiogenesis is crucial. Therefore, targeting local or cell-specific ADAMTS7 knockdown shows promise as a therapeutic strategy for managing atherosclerosis-related risks.

### 2.4 Enhancing efficacy and reducing toxicity: Synergistic approach of low-concentration siRNA combinations

The utilization of a combination of distinct siRNA molecules at lower concentrations presents a promising strategy to enhance efficacy while simultaneously reducing the potential for toxicity and off-target effects. In this study, we explored various combinations of the three lead siRNAs to evaluate their potential for effective gene knockdown. Additionally, employing reduced concentrations of individual siRNAs alleviates the overall cellular burden, potentially mitigating unintended interactions and adverse cellular responses. Overall, all three lead siRNAs demonstrated strong knockdown as shown above below 10% each using 10 nM siRNA (Figure 10). After that we tested different combinations. siRNA 5 and 6 are preferably not used in combination due to high sequence similarity. In line and as shown in Figure 10 they performed moderately when combined and compared to other combinations. Therefore, our findings suggest that the combination of siRNA phuADAMTS7-6 and -7 (5% ± 1.5%), at low concentration each (5 nM), proves to be an optimal strategy, offering a more precise and safer approach to achieve efficient gene suppression while mitigating undesired side effects.

### 2.5 Efficient Transduction in Human Smooth Muscle Cells Using AAV2 Serotypes

In our quest for effective gene therapies, we are exploring not only the non-viral delivery of siRNA but also actively investigating the use of adeno-associated viruses (AAVs) for delivery. Our primary goal is to design a potent gene therapy for ADAMTS7 knockdown, utilizing shRNA with cross-species target sequences identified in Table 1. AAV-2 serotypes, as illustrated in Figure 11, have shown superior transduction efficiency in human smooth muscle cells (huSMCs).

To increase knockdown efficiency, we will use either a combination of four shRNAs (phu -1, -2, -6, -7) or an alternative set (phu -1, -2, -5, -7). Notably, two siRNAs (phu-siADAMTS7 -5 and -6) targeting the same sequence with a 2-base pair displacement will be incorporated. Our vector design (pAAV-4miR30; Figure 12; VectorBuilder) encompasses four lead siRNAs, that will be packaged into the AAV-2 serotypes. The forthcoming evaluation will focus on the knockdown efficiency and biological impact of this vector on ADAMTS7, specifically assessing huSMC proliferation and migration.

### 2.6 Utilizing AAVs in Stent Coating for Restenosis Prevention

Stent-based gene therapy, particularly involving AAVs, offers a promising avenue for delivering therapeutic genes directly to target tissue sites. This strategy includes the reversible immobilization of AAV2 vectors on metal substrates and coating stents with AAVs carrying shRNA sequences. Our approach is guided by a recently reported protocol by Hooshdaran et al., 2022 or will be adapted to other state-of-the art coating stents or balloons approach.

To optimize viral vector concentration for effective gene delivery, we will utilize an *ex-vivo* assay in a preclinical Minipig model carotid setting. Molecular assays, including qPCR and Western blot analysis, will monitor the expression of therapeutic shRNAs, assess the inhibition of ADAMTS7 genes, and evaluate the overall efficacy of the AAV-shRNA-coated stent. An *in-vivo* validation in Minipig will be conducted as well.

In conclusion, the application of ADAMTS7 knockdown holds immense promise, not only for restenosis and cardiovascular diseases but also for addressing the complexities of other pathogenic processes, including inflammatory arthritis, thoracic aortic aneurysm and dissection, asthma, psoriasis, COPD, and lung cancer. These insights pave the way for innovative therapeutic strategies that may revolutionize the treatment landscape for these diverse diseases.

### References:

Mohsen Naghavi et al. Global, regional, and national age-sex specific mortality for 264 causes of death, 1980-2016: a systematic analysis for the Global Burden of Disease Study 2016. Lancet 390, 1151-1210 (2017).
Roth, G.A. et al. Global Burden of Cardiovascular Diseases and Risk Factors, 1990-2019: Update From the GBD 2019 Study. J Am Coll Cardiol 76, 2982-3021 (2020).
Kim, M.S. & Dean, L.S. In-stent restenosis. Cardiovasc Ther 29, 190-8 (2011).
McPherson, R. & Tybjaerg-Hansen, A. Genetics of Coronary Artery Disease. Circ Res 118, 564-78 (2016).
Erdmann, J., Kessler, T., Munoz Venegas, L. & Schunkert, H. A decade of genome-wide association studies for coronary artery disease: the challenges ahead. Cardiovasc Res 114, 1241-1257 (2018).
Erdmann, J. et al. Dysfunctional nitric oxide signalling increases risk of myocardial infarction. Nature 504, 432-6 (2013).
Erdmann, J. et al. Genome-wide association study identifies a new locus for coronary artery disease on chromosome 10p11.23. Eur Heart J 32, 158-68 (2011).
Schunkert, H. et al. Large-scale association analysis identifies 13 new susceptibility loci for coronary artery disease. Nat Genet 43, 333-8 (2011).
Myocardial Infarction Genetics, C. et al. Genome-wide association of early-onset myocardial infarction with single nucleotide polymorphisms and copy number variants. Nat Genet 41, 334-41 (2009).
Erdmann, J. et al. New susceptibility locus for coronary artery disease on chromosome 3q22.3. Nat Genet 41, 280-2 (2009).
Samani, N.J. et al. Genomewide association analysis of coronary artery disease. N Engl J Med 357, 443-53 (2007).
Koyama, S. et al. Population-specific and trans-ancestry genome-wide analyses identify distinct and shared genetic risk loci for coronary artery disease. Nat Genet 52, 1169-1177 (2020).
Wang, L. et al. ADAMTS-7 mediates vascular smooth muscle cell migration and neointima formation in balloon-injured rat arteries. Circ Res 104, 688-98 (2009).
Qin, W., Cao, Y., Li, L., Chen, W. & Chen, X. Upregulation of ADAMTS7 and downregula-tion of COMP are associated with aortic aneurysm. Mol Med Rep 16, 5459-5463 (2017).
Kessler, T. et al. ADAMTS-7 inhibits re-endothelialization of injured arteries and promotes vascular remodeling through cleavage of thrombospondin-1. Circulation 131, 1191-201 (2015).
van Setten, J. et al. Genome-wide association study of coronary and aortic calcification implicates risk loci for coronary artery disease and myocardial infarction. Atherosclerosis 228, 400-5 (2013).
Bauer, R.C. et al. Knockout of Adamts7, a novel coronary artery disease locus in humans, reduces atherosclerosis in mice. Circulation 131, 1202-1213 (2015).
Gong, Z. et al. ADAMTS-7 deficiency attenuates thoracic aortic aneurysm and dissection in mice. J Mol Med (Berl) (2023).
Gailus-Durner, V. et al. Introducing the German Mouse Clinic: open access platform for standardized phenotyping. Nat Methods 2, 403-4 (2005).
Kelwick, R., Desanlis, I., Wheeler, G.N. & Edwards, D.R. The ADAMTS (A Disintegrin and Metalloproteinase with Thrombospondin motifs) family. Genome Biol 16, 113 (2015).
Pecin, I., Hartgers, M.L., Hovingh, G.K., Dent, R. & Reiner, Z. Prevention of cardiovascular disease in patients with familial hypercholesterolaemia: The role of PCSK9 inhibitors. Eur J Prev Cardiol 24, 1383-1401 (2017).
Ge, X. et al. A Systematic Review and Meta-Analysis of Therapeutic Efficacy and Safety of Alirocumab and Evolocumab on Familial Hypercholesterolemia. Biomed Res Int 2021, 8032978 (2021).
Lopez-Melgar, B. et al. Short-Term Progression of Multiterritorial Subclinical Atherosclerosis. J Am Coll Cardiol 75, 1617-1627 (2020).
Zhang, L. et al. Identification of cardiovascular health gene variants related to longevity in a Chinese population. Aging (Albany NY) 12, 16775-16802 (2020).
Reilly, M.P. et al. Identification of ADAMTS7 as a novel locus for coronary atherosclerosis and association of ABO with myocardial infarction in the presence of coronary atherosclerosis: two genome-wide association studies. Lancet 377, 383-92 (2011).
Ma, W.F. et al. Enhanced single-cell RNA-seq workflow reveals coronary artery disease cellular cross-talk and candidate drug targets. Atherosclerosis 340, 12-22 (2022).
Pu, X. et al. ADAMTS7 cleavage and vascular smooth muscle cell migration is affected by a coronary-artery-disease-associated variant. Am J Hum Genet 92, 366-74 (2013).
Donovan, D., Brown, N.J., Bishop, E.T. & Lewis, C.E. Comparison of three in vitro human 'angiogenesis' assays with capillaries formed in vivo. Angiogenesis 4, 113-21 (2001).
Hooshdaran, B. et al. Stent-based delivery of AAV2 vectors encoding oxidation-resistant apoA1. Sci Rep 12, 5464 (2022).
Liu, C.J. et al. ADAMTS-7: a metalloproteinase that directly binds to and degrades cartilage oligomeric matrix protein. FASEB J 20, 988-90 (2006).
Zhang, Y., Wei, F. & Liu, C.J. Overexpression of ADAMTS-7 leads to accelerated initiation and progression of collagen-induced arthritis in mice. Mol Cell Biochem 404, 171-9 (2015).
Loyola, K., Karsulovic, C., Cabrera, R., Perez, C. & Hojman, L. New Markers for Cardiovascular Disease in Psoriatic Patients: Preliminary Study on Monocyte Phenotype, ADAMTS7, and mTOR Activity. Metabolites 13(2023).
Karsulovic, C., Loyola, K., Cabrera, R., Perez, C. & Hojman, L. Non-Canonical WNT/Wnt5a Pathway Activity in Circulating Monocytes of Untreated Psoriatic Patients: An Exploratory Study of Its Association with Inflammatory Cytokines and Cardiovascular Risk Marker-ADAMTS7. Biomedicines 11(2023).
Jaiswal, A.K. & Mishra, A. ADAMTS7 Attenuates House Dust Mite-Induced Airway Inflammation and Th2 Immune Responses. Lung 200, 305-313 (2022).
Bauer, C. et al. Etidronate prevents dystrophic cardiac calcification by inhibiting macrophage aggregation. Sci Rep 8, 5812 (2018).
Libby, P., Ridker, P. M., & Hansson, G. K. (2011). Progress and Challenges in Translating the Biology of Atherosclerosis. Nature, 473(7347), 317-325.
Lusis, A. J. (2000). Atherosclerosis. Nature, 407(6801), 233-241.
Virmani, R. et al. (2000). Lessons from Sudden Coronary Death: A Comprehensive Morphological Classification Scheme for Atherosclerotic Lesions. Arterioscler Thromb Vasc Biol, 20(5), 1262-1275.
Ference, B. A. et al. (2017). LowDensity Lipoproteins Cause Atherosclerotic Cardiovascular Disease. 1. Evidence from Genetic, Epidemiologic, and Clinical Studies. A Consensus Statement from the European Atherosclerosis Society Consensus Panel. Eur Heart J, 38(32), 2459-2472.
Rassow, J. et al. (2012). Duale Reihe Biochemie, 3. Auflage. Stuttgart: Thieme.
Catalanotto C, Cogoni C, Zardo G. MicroRNA in Control of Gene Expression: An Overview of Nuclear Functions. Int J Mol Sci. 2016 Oct 13;17(10):1712. doi: 10.3390/ijms17101712. PMID: 27754357; PMCID: PMC5085744.
Du Q, Thonberg H, Wang J, Wahlestedt C, Liang Z. A systematic analysis of the silencing effects of an active siRNA at all single-nucleotide mismatched target sites. Nucleic Acids Res. 2005;33:1671-1677.
Ahmed F, Raghava GP. Designing of highly effective complementary and mismatch siRNAs for silencing a gene. PLoS One. 2011;6(8):e23443.
Selvam C, Mutisya D, Prakash S, Ranganna K, Thilagavathi R. Therapeutic potential of chemically modified siRNA: Recent trends. Chem Biol Drug Des. 2017 Nov;90(5):665-678. doi: 10.1111/cbdd. 12993. Epub 2017 May 16. PMID: 28378934; PMCID: PMC5935465.
Brown KM, Nair JK, Janas MM, Anglero-Rodriguez Yl, Dang LTH, Peng H, Theile CS, Cas-tellanos-Rizaldos E, Brown C, Foster D, Kurz J, Allen J, Maganti R, Li J, Matsuda S, Stricos M, Chickering T, Jung M, Wassarman K, Rollins J, Woods L, Kelin A, Guenther DC, Mobley MW, Petrulis J, McDougall R, Racie T, Bombardier J, Cha D, Agarwal S, Johnson L, Jiang Y, Lentini S, Gilbert J, Nguyen T, Chigas S, LeBlanc S, Poreci U, Kasper A, Rogers AB, Chong S, Davis W, Sutherland JE, Castoreno A, Milstein S, Schlegel MK, Zlatev I, Charisse K, Keat-ing M, Manoharan M, Fitzgerald K, Wu JT, Maier MA, Jadhav V. Expanding RNAi therapeutics to extrahepatic tissues with lipophilic conjugates. Nat Biotechnol. 2022 Oct;40(10):1500-1508. doi: 10.1038/s41587-022-01334-x. Epub 2022 Jun 2. PMID: 35654979.
Tarn WY, Cheng Y, Ko SH, Huang LM. Antisense Oligonucleotide-Based Therapy of Viral Infections. Pharmaceutics. 2021 Nov26;13(12):2015. doi: 10.3390/pharmaceutics13122015. PMID: 34959297; PMCID: PMC8707165.
Raouane M, Desmaële D, Urbinati G, Massaad-Massade L, Couvreur P. Lipid conjugated oligonucleotides: a useful strategy for delivery. Bioconjug Chem. 2012 Jun 20;23(6):1091-104. doi: 10.1021/bc200422w. Epub 2012 Mar 15. PMID: 22372953. https://en.wikipedia.org/wiki/Stent
Hossfeld S, Nolte A, Hartmann H, Recke M, Schaller M, Walker T, Kjems J, Schlosshauer B, Stoll D, Wendel HP, Krastev R. Bioactive coronary stent coating based on layer-by-layer technology for siRNA release. Acta Biomater. 2013 May;9(5):6741-52. doi: 10.1016/j.actbio.2013.01.013. Epub 2013 Jan 17. PMID: 23333865.
Nolte A, Walker T, Schneider M, Kray O, Avci-Adali M, Ziemer G, Wendel HP. Small-interfering RNA-eluting surfaces as a novel concept for intravascular local gene silencing. Mol Med. 2011;17(11-12):1213-22. doi: 10.2119/molmed.2011.00143. Epub 2011 Jul 22. PMID: 21792480; PMCID: PMC3321820.
Che HL, Bae IH, Lim KS, Song IT, Lee H, Lee D, Kim WJ, Jeong MH, Ahn Y. Therapeutic Effect of Akt1 siRNA Nanoparticle Eluting Coronary Stent on Suppression of Post-Angioplasty Restenosis. J Biomed Nanotechnol. 2016 Jun;12(6):1211-22. doi: 10.1166/jbn.2016.2255. PMID: 27319215.
Koenig O, Nothdurft D, Perle N, Neumann B, Behring A, Degenkolbe I, Walker T, Schlensak C, Wendel HP, Nolte A. An Atelocollagen Coating for Efficient Local Gene Silencing by Using Small Interfering RNA. Mol Ther Nucleic Acids. 2017 Mar 17;6:290-301. doi: 10.1016/j.omtn.2017.01.006. Epub 2017 Feb 9. PMID: 28325296; PMCID: PMC5363512.
Aurélie San Juan, Madiha Bala, Hanna Hlawaty, Patrick Portes, Roger Vranckx, Laurent J. Feldman, and Didier Letourneur, Development of a Functionalized Polymer for Stent Coating in the Arterial Delivery of Small Interfering RNA. Biomacromolecules 2009 10 (11), 3074-3080, DOI: 10.1021/bm900740g.
Joddar B, Albayrak A, Kang J, Nishihara M, Abe H, Ito Y. Sustained delivery of siRNA from dopamine-coated stainless steel surfaces. Acta Biomater. 2013 May;9(5):6753-61. doi: 10.1016/j.actbio.2013.01.007. Epub 2013 Jan 16. Erratum in: Acta Biomater. 2014 Aug;10(8):3811. PMID: 23333442.
Wang Y, Li M, Sheng Z, Ran H, Dong J, Fang L, Zhang P. Ultrasound-mediated delivery of Pik3cb shRNA using magnetic nanoparticles for the treatment of in-stent restenosis in a rat balloon-injured model. J Radiat Res. 2024 Jan 19;65(1):47-54. doi: 10.1093/jrr/rrad083. PMID: 37948449; PMCID: PMC10803161.
G. Decher, J.D. Hong, J. Schmitt, Buildup of ultrathin multilayer films by a self-assembly process: III. Consecutively alternating adsorption of anionic and cationic polyelectrolytes on charged surfaces, Thin Solid Films, Volumes 210-211, Part 2, 1992, Pages 831-835, ISSN 0040-6090, https://doi.org/10.1016/0040-6090(92)90417-A.
   WO2012045469A1
Fishbein I, Guerrero DT, Alferiev IS, Foster JB, Minutolo NG, Chorny M, Monteys AM, Driesbaugh KH, Nagaswami C, Levy RJ. Stent-based delivery of adeno-associated viral vectors with sustained vascular transduction and iNOS-mediated inhibition of in-stent restenosis. Gene Ther. 2017 Nov;24(11):717-726. doi: 10.1038/gt.2017.82. Epub 2017 Aug 23. PMID: 28832561; PMCID: PMC5709213.
Charles Landau, Mark J. Pirwitz, Maureen A. Willard, Robert D. Gerard, Robert S. Meidell, John E. Willard, Adenoviral mediated gene transfer to atherosclerotic arteries after balloon angioplasty, American Heart Journal, Volume 129, Issue 6, 1995, Pages 1051-1057, ISSN 0002-8703, https://doi.org/10.1016/0002-8703(95)90383-6.
   https://mediatum.ub.tum.de/doc/1543434/1543434.pdf

## Claims

1. An inhibitory oligonucleotide capable of selectively binding to a target nucleic acid sequence within an mRNA encoding *ADAMTS7,* wherein the target nucleic acid sequence is selected from the group consisting of SEQ ID NO: 1, SEQ ID NO: 2, SEQ ID NO: 3, SEQ ID NO: 4, SEQ ID NO: 5, SEQ ID NO: 6 and SEQ ID NO: 7, wherein the inhibitory oligonucleotide molecule is selected from the group consisting of an RNA molecule capable of inducing RNAi and an antisense oligonucleotide (ASO).

2. The inhibitory oligonucleotide of claim 1, wherein the target nucleic acid sequence is conserved in human, pig and mouse and is selected from the group consisting of SEQ ID NO: 1, 2 and 3.

3. The inhibitory oligonucleotide of any of claims 1 or 2, wherein the target nucleic acid sequence is SEQ ID NO: 1.

4. The inhibitory oligonucleotide of claim 1, wherein the oligonucleotide molecule is an RNA molecule capable of inducing RNAi selected from the group comprising an siRNA or an shRNA, wherein, optionally, the RNA molecule capable of inducing RNAi comprises a nucleic acid sequence having at least 90% sequence identity to any of SEQ ID NO: 8, SEQ ID NO: 10, SEQ ID NO: 12, SEQ ID NO: 14, SEQ ID NO: 16, SEQ ID NO: 18 or SEQ ID NO: 20, preferably to any of SEQ ID NO: 8, SEQ ID NO: 10, or SEQ ID NO: 12.

5. The inhibitory oligonucleotide of claim 1, wherein the oligonucleotide is an antisense oligonucleotide (ASO) comprising a nucleic acid sequence that corresponds to a subsequence of at least 6 nucleotides of any of SEQ ID NO: 8, SEQ ID NO: 10, SEQ ID NO: 12, SEQ ID NO: 14, SEQ ID NO: 16, SEQ ID NO: 18 or SEQ ID NO: 20, preferably of SEQ ID NO: 8, SEQ ID NO: 10, or SEQ ID NO: 12.

6. A composition providing one or more of the inhibitory oligonucleotide molecules according to any of the preceding claims.

7. The composition of claim 6, wherein the composition comprises one or more inhibitory oligonucleotides according to any of claims 1-5, wherein, optionally, the composition further comprises a delivery vehicle that encloses and/or is associated with the one or more inhibitory oligonucleotides.

8. The composition of claim 6, wherein the one or more inhibitory oligonucleotides are shRNA and the composition comprises a delivery vehicle configured to express the shRNA from a nucleic acid encoding said shRNA.

9. The composition of claim 8, wherein the delivery vehicle is a viral vector, wherein, preferably, the viral vector is an AAV vector of serotype AAV-2.

10. The composition of any of claims 6-9, wherein the composition is a pharmaceutical composition, optionally further comprising a pharmaceutically acceptable carrier and/or an inhibitory oligonucleotide capable of inhibiting PCSK9, e.g., Inclirisan and/or an inhibitory oligonucleotide capable of inhibiting apolipoprotein B-100, e.g., Mipomersen.

11. An implantable medical device, wherein the implantable medical device comprises and is capable of releasing one or more inhibitory oligonucleotides according to any of claims 1-5 or a composition according to any of claims 6-10, and wherein, optionally, the implantable medical device is a stent or a balloon catheter.

12. The implantable medical device of claim 11, wherein the device is coated with the one or more inhibitory oligonucleotides according to any of claims 1-5 or a composition according to any of claims 6-10.

13. The inhibitory oligonucleotide of any of claims 1-5, the composition of any of claims 6-10, or the medical device of any of claims 11 or 12 for use in a method of treating a cardiovascular disease or disorder selected from the group consisting of atherosclerosis and atherosclerosis-induced neovascularization, restenosis, coronary artery disease (CAD) and thoracic aortic aneurysm and dissection (TAAD), wherein, optionally, the treatment is combined with a HMG-CoA reductase inhibitor selected from the group of statins comprising atorvastatin, fluvastatin, lovastatin, pitavastatin, pravastatin, rosu-vastain and simvastatin, and/or a PCSK9 inhibitor selected from the group comprising an antibody to PCSK9 and an inhibitory oligonucleotide targeting PCSK9 such as inclirisan and/or an inhibitor of apolipoprotein B-100 such as Mipomersen.

14. The inhibitory oligonucleotides of any of claims 1-5, the composition of any of claims 6-10, or the medical device of any of claims 11 or 12 for use in a method of treating an inflammatory disease selected from the group comprising inflammatory arthritis and psoriasis.

15. The inhibitory oligonucleotide of any of claims 1-5 or the composition of any of claims 6-10, for use in a method of treating a pulmonary disease selected from the group consisting of asthma, chronic obstructive pulmonary disease (COPD) and lung cancer.
